(19) Europäisches Patentamt
European Patent Office
Office européen des brevets

(11) EP 4 308 919 B1

(12) **EUROPÄISCHE PATENTSCHRIFT**

(45) Veröffentlichungstag und Bekanntmachung des Hinweises auf die Patenterteilung:
**24.12.2025 Patentblatt 2025/52**

(21) Anmeldenummer: **22717078.4**

(22) Anmeldetag: **18.03.2022**

(51) Internationale Patentklassifikation (IPC):
***G01N 31/22*** *(2006.01)* ***G01N 27/06*** *(2006.01)*
***G01N 33/18*** *(2006.01)* ***G05D 21/00*** *(2006.01)*
***G01N 21/77*** *(2006.01)* ***G01N 21/78*** *(2006.01)*

(52) Gemeinsame Patentklassifikation (CPC):
**G01N 31/22; G01N 21/77; G01N 33/18;**
G01N 21/78; G01N 2021/7783

(86) Internationale Anmeldenummer:
**PCT/EP2022/057206**

(87) Internationale Veröffentlichungsnummer:
**WO 2022/195093 (22.09.2022 Gazette 2022/38)**

(54) **ANALYSESYSTEM ZUR BESTIMMUNG VON IONEN IN EINEM IONENHALTIGEN FLÜSSIGEN MEDIUM UND ANALYSEVERFAHREN**

ANALYSIS SYSTEM FOR THE DETERMINATION OF IONS IN AN ION-CONTAINING LIQUID MEDIUM, AND ANALYSIS METHOD

SYSTÈME D’ANALYSE POUR LA DÉTERMINATION DES IONS DANS UN MILIEU LIQUIDE CONTENANT DES IONS, PROCÉDÉ D’ANALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.03.2021 DE 102021001442**

(43) Veröffentlichungstag der Anmeldung:
**24.01.2024 Patentblatt 2024/04**

(73) Patentinhaber: **Dimetamus UG (haftungsbeschränkt)**
**77866 Rheinau (DE)**

(72) Erfinder: **HAMM, Lukas-David**
**77866 Rheinau (DE)**

(74) Vertreter: **Patentanwälte Bauer Vorberg Kayser Partnerschaft mbB**
**Goltsteinstraße 87**
**50968 Köln (DE)**

(56) Entgegenhaltungen:
**EP-A1- 1 167 954**
**JP-A- S 579 864**
**JP-A- S5 858 459**
**US-A- 5 788 828**
**US-A1- 2013 220 814**

EP 4 308 919 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Analysesystem zur qualitativen und/oder quantitativen Bestimmung von Ionen in einem ionenhaltigen flüssigen Medium. Ebenso betrifft die vorliegende Erfindung ein mit dem genannten Analysesystem ausgeführtes Analyseverfahren.

Um ionische Parameter in wässriger Lösung zu bestimmen sowie automatisch mittels Sonden überwachen zu können, werden heutzutage häufig ionenselektive Sonden eingesetzt. Diese können jeweils einen ionischen Parameter über dessen Redoxpotential in der Lösung bestimmen. Die Ionenselektivität wird hierbei durch den Einsatz ionenselektiver Membranen erreicht.

Diese Sonden zeigen in der Praxis allerdings einige gravierende Nachteile. So treten bei den Elektroden Querempfindlichkeiten auf. Dies bedeutet, dass die Elektroden in der Regel keine vollständige Selektivität aufweisen und somit auch Störionen mitmessen, was die Messergebnisse verfälscht. Ein weiteres Problem ist die hohe Empfindlichkeit der eingesetzten Membranen gegen Umwelteinflüsse. Zudem sind ionenselektiven Sonden teuer, was sie für bestimmte (beispielsweise private) Anwender unzugänglich macht. Letztlich müssen derartige Sonden regelmäßig neu kalibriert werden, was vor allem bei einem Dauereinsatz zu einen hohen Wartungsaufwand führt.

Die zunehmende Verschmutzung von Gewässern ist ein globales Problem und auch in technologisch hochentwickelten Ländern der ersten Welt sehr präsent. Eine Ursache dieses Problems ist die fortschreitende Industrialisierung und Kommerzialisierung des Agrarsektors. So muss die Agrarwirtschaft, um den weltweiten Nahrungsbedarf zu decken, immer mehr Nahrungsmittel auf immer geringerer Fläche erzeugen. Zusätzlich kommt hierbei die steigende Nachfrage nach Biomasse für die klimaneutrale Energieerzeugung erschwerend hinzu. Um diesen Bedarf decken zu können, muss die Landwirtschaft hierbei auf künstliche Düngemittel zurückgreifen, welche die Bodenflächen auch bei einer sehr hohen Beanspruchung fruchtbar halten. Zudem lässt sich durch den Einsatz von Düngemitteln das Wachstum der Nutzpflanzen und somit auch der gesamte Ertrag steigern. Allerdings werden die eingesetzten Düngemittel nicht vollständig von den Nutzpflanzen aufgenommen und der Düngemittelüberschuss wird durch Regen in die umliegenden Gewässer und in das Grundwasser eingetragen, was schwerwiegende Folgen für die dort vorkommenden Ökosysteme haben kann.

So führt ein übermäßiges Vorkommen von pflanzlichen Nährstoffen zu einer vermehrten Bildung von Algen. Abgestorbene Algen sinken zum Gewässerboden und werden dort in der Regel durch aerobe Abbauprozesse verwertet. Kommt es nun zu einem übermäßigen Algenwachstum, dann sterben auch mehr Algen ab und zum Abbau dieser Biomasse wird mehr Sauerstoff benötigt. Dies kann wiederum tödlich für weitere im Gewässer vorkommenden Lebewesen, wie Fische, wirken und beispielsweise ein massenhaftes Fischsterben auslösen. Ist mit der Zeit kein Sauerstoff mehr im Gewässer vorhanden, dann erfolgt der Biomasseabbau anaerob, also ohne Sauerstoff, was zusätzlich die Bildung schädlicher Faulgase zur Folge hat und umgangssprachlich als das "Umkippen" des Gewässers bezeichnet wird.

Eine Überdüngung ist allerdings nicht nur schädlich für die betroffenen Ökosysteme, sondern kann auch dem Menschen direkt schaden. So kann der in den Düngemitteln enthaltenen Stickstoff als Nitrat in das Grundwasser gelangen und von dort in unser Trinkwasser. In unserem Körper in Nitrit umgewandelt, kann das Nitrat wiederum gesundheitliche Schäden zur Folge haben.

Um einer Überdüngung zuvorkommen zu können, muss eine ständige Überwachung der Gewässer durchgeführt werden. So könnte die Düngung an sich optimiert werden, aber auch eine schnelle Reaktion auf zu hohe Düngemittelkonzentrationen wäre möglich. Allerdings sind die aktuell genutzten Verfahren nicht zur automatischen und großflächigen Gewässerüberwachung geeignet. Es ist zudem von Interesse, die Düngemittelkonzentrationen in Echtzeit zu überwachen und die Düngemittelzugabe vollautomatisch zu regeln. Allerdings sind aktuelle automatische Messsysteme und Verfahren für eine solche Anwendung zu teuer, wartungsintensiv und störanfällig.

Ferner ist die Überwachung von Ionengehalten sowie die Identifizierung bestimmter Ionenarten in flüssigen System auch in anderen Anwendungsfeldern von Interesse, beispielsweise in Wasseraufbereitungsanlagen, Kläranlagen, Industrieanlagen etc. Ein weiteres relevantes Anwendungsgebiet für die Überwachung von Ionengehalten betrifft die Aquaristik, d. h. die qualitative und/oder qualitative Bestimmung von Ionen in ionenhaltigen flüssigen Medien, die in Aquarien eingesetzt werden. Gerade in Aquarien werden häufig Pflanzenarten und Tierarten (Fische, Kleintiere etc.) gehalten, die empfindlich gegenüber bestimmten Umweltbedingungen (z. B. auch den Ionengehalt im Wasser des Aquariums) sind. Eine zuverlässige und einfach handhabbare Überwachungsmethode der dort vorliegenden Ionenarten in qualitativer und quantitativer Hinsicht ist daher von großem Interesse.

Aufgabe der Erfindung ist demnach die Entwicklung eines Analysesystems und eines Analyseverfahrens zur Bestimmung von Ionen in einem ionenhaltigen flüssigen Medium mit dem die Nachteile der im Stand der Technik eingesetzten Analysesysteme und -verfahren umgangen werden. So soll das erfindungsgemäß vorgeschlagene Analysesystem bzw. Analyseverfahren im Vergleich zu aus dem Stand der Technik bekannte Systemen kostengünstiger herstellbar respektive betreibbar sein. Weiterhin soll das Analysesystem bzw. Analyseverfahren unempfindlicher gegenüber Stör-Ionen sein und einen geringen Wartungsaufwand erfordern. Zudem soll das Analysesystem bzw. das Analyseverfahren einfach handhabbar sein. Ein elektrochemisches Analysesystem für ionenhaltige Flüssigkeiten ist aus

dem japanischen Patent JPS579864 A bekannt.

Zur Lösung dieser Aufgabe wird ein Analysesystem mit den Merkmalen des Patentanspruchs 1 sowie ein Analyseverfahren mit den Merkmalen des Patentanspruchs 13 vorgeschlagen.

Wie bereits erwähnt, betrifft die vorliegende Erfindung ein Analysesystem zur Bestimmung von Ionen in einem ionenhaltigen flüssigen Medium.

Unter einem ionenhaltigen flüssigen Medium kann dabei Materie in einem flüssigen Aggregatzustand verstanden werden, die Ionen enthalten kann. Das flüssige ionenhaltige Medium kann beispielsweise eine ionenhaltige Flüssigkeit, z. B. eine flüssige Lösung, sein. Eine ionenhaltige flüssige Lösung kann dabei als ein homogenes Gemisch verstanden werden, das in einem Lösungsmittel gelöste Ionen mindestens einer Ionenart umfasst. Dabei kann das Lösungsmittel beispielsweise Wasser, und die Lösung dementsprechend eine wässrige Lösung sein. Das zu untersuchende ionenhaltige flüssige Medium kann beispielsweise eine aus einem statischen oder fließenden Gewässer, wie einem See oder einem Fluss, entnommene Probe sein. Gleichsam kann das zu untersuchende ionenhaltige flüssige Medium aus einem statischen oder fließenden Reservoir einer Kläranlage, einer Wasseraufbereitungsanlage, einem Aquarium oder einer Industrieanlage stammen.

Ionen können dabei grundsätzlich als elektrisch geladene Teilchen verstanden werden, die aus ungeladenen Atomen oder Molekülen durch die Abgabe oder Anlagerung von Elektronen entstehen können. Kationen können dabei als positiv geladene Ionen verstanden werden, welche aus ungeladenen Atomen oder Molekülen durch die Abgabe von Elektronen entstehen. Anionen wiederum können als negativ geladene Ionen verstanden werden, welche aus ungeladenen Atomen oder Molekülen durch die Anlagerung von Elektronen entstehen. Anionen und Kationen können ein- oder mehrwertig geladen sein.

Die Bestimmung der Ionen erfolgt dabei grundsätzlich qualitativ und/oder quantitativ. Die Bestimmung der Ionen kann also sowohl qualitativ als auch quantitativ erfolgen. Alternativ dazu kann die Bestimmung der Ionen nur quantitativ, oder nur qualitativ erfolgen. Unter einer "quantitativen" Bestimmung ist im Kontext der vorliegenden Offenbarung eine mengenmäßige Bestimmung zu verstehen, beispielsweise einer Menge (auch Stoffmenge) pro Volumen, einer absoluten Menge (auch Stoffmenge, einer Masse oder dergleichen), einer relativen Menge in Bezug zu einem Vergleichsmaß, oder dergleichen. Unter einer "qualitativen" Bestimmung ist hingegen eine Klassifizierung nach Art/Typ einer Ionenspezies zu verstehen, beispielsweise hinsichtlich der Frage, ob eine bestimmte Ionenart vorliegt oder nicht.

Das vorgeschlagene Analysesystem zur Bestimmung der in dem ionenhaltigen flüssigen Medium vorliegenden Ionen umfasst eine Messzelle und eine Messanordnung.

Dabei umfasst die Messzelle des Analysesystems eine erste Messkammer, eine zweite Messkammer, sowie eine zwischen der ersten Messkammer und der zweiten Messkammer angeordnete Probenkammer, die zur Aufnahme des ionenhaltigen flüssigen Mediums ausgebildet ist. Dabei ist in der ersten Messkammer eine erste Elektrode und in der zweiten Messkammer eine zweite Elektrode angeordnet.

Dabei kann unter einer Elektrode grundsätzlich jeder elektrische Leiter in Form eines Feststoffes verstanden werden, der in der Lage ist, elektrische Ladungsträger zu leiten. Unter einem "Leiten" kann dabei ein Transport von elektrischen Ladungsträgern, wie Elektronen oder Ionen, verstanden werden. Elektronen können dabei als elektrisch geladene Elementarteilchen verstanden werden, die eine negative Ladung aufweisen. Die Elektrode kann dabei aus einem leitenden Material, beispielsweise aus Graphit, oder einem Metall wie Titan oder Platin gefertigt sein. An einer Elektrode können dabei elektrische Ladungsträger von dem Feststoff, aus dem die Elektrode gefertigt ist, auf ein das die Elektrode umgebendes Medium übergehen, oder elektrische Ladungsträger aus besagtem Medium aufgenommen werden.

Zwischen der ersten und zweiten Elektrode kann dabei eine Spannung so angelegt werden, dass die erste Elektrode positiv und die zweite Elektrode negativ polarisiert (geladen) wird, wodurch die Anionen in Richtung der ersten Elektrode und die Kationen in Richtung der zweiten Elektrode befördert werden. Unter einer "Beförderung" von Ionen kann dabei beispielsweise ein Ionentransport in einem flüssigen Medium zu verstehen sein.

Zur Erzeugung einer elektrischen Spannung zwischen der ersten und der zweiten Elektrode kann grundsätzlich jede elektrische Spannungsquelle eingesetzt werden, die dazu ausgebildet ist, eine elektrische Spannung zwischen zwei Elektroden zu erzeugen. Unter einer elektrischen Spannungsquelle kann ein aktiver Zweipol verstanden werden, der zwischen seinen Anschlusspunkten eine elektrische Spannung erzeugt. Unter einem Zweipol kann dabei ein elektrisches Bauelement oder eine elektrische Schaltung mit zwei Anschlüssen (Klemmen, Polen) verstanden werden. Die elektrische Spannungsquelle kann beispielsweise eine Batterie oder ein Generator sein. Zur Erzeugung einer Spannung zwischen der ersten und der zweiten Elektrode kann die elektrische Spannungsquelle über einen ersten elektrischen Leiter mit der ersten Elektrode und einen zweiten elektrischen Leiter mit der zweiten Elektrode verbunden sein. Entsprechend der elektrischen Polarität zwischen der ersten und der zweiten Elektrode kann an der ersten respektive zweiten Elektrode entweder ein Elektronenmangel oder ein Elektronenüberschuss herrschen. Die Elektrode kann dementsprechend positiv oder negativ polarisiert werden.

Zudem ist zwischen der ersten Messkammer und der Probenkammer eine anionenselektive Membran angeordnet, die dazu ausgebildet ist, einen Durchtritt von Anionen aus der Probenkammer in die erste Messkammer zu

ermöglichen.

Weiterhin ist zwischen der zweiten Messkammer und der Probenkammer eine kationenselektive Membran angeordnet, die dazu ausgebildet ist, einen Durchtritt von Kationen aus der Probenkammer in die zweite Messkammer zu ermöglichen.

Die erste Messkammer ist mit einem ersten flüssigen Messkammermedium befüllt. Dabei kann das erste flüssige Messkammermedium einen ersten Erkennungsstoff enthalten, um bei Durchtritt von Anionen in die erste Messkammer Anionen-Erkennungsstoff-Assoziate zu bilden. Zudem ist die zweite Messkammer mit einem zweiten flüssigen Messkammermedium befüllt. Dabei kann das zweite flüssige Messkammermedium einen zweiten Erkennungsstoff enthalten, um bei Durchtritt von Kationen in die zweite Messkammer Kationen-Erkennungsstoff-Assoziate zu bilden.

Im Zusammenhang mit der vorliegenden Erfindung kann dabei unter einem flüssigen Messkammermedium jedes in einem flüssigen Aggregatzustand vorliegende Medium verstanden werden, mit dem eine Messkammer befüllt werden kann. Dabei kann das erste flüssige Messkammermedium als ein solches flüssiges Messkammermedium verstanden werden, mit dem die erste Messkammer befüllt wird. Das zweite flüssige Messkammermedium kann wiederum als ein solches flüssiges Messkammermedium verstanden werden, mit dem die zweite Messkammer befüllt wird. Dabei kann das erste flüssige Messkammermedium sowie das zweite flüssige Messkammermedium dem ionenhaltigen flüssigen Medium entsprechen. Unter einer "Befüllung" kann eine vollständige Befüllung der jeweiligen Messkammer mit dem jeweiligen flüssigen Messkammermedium verstanden werden, gleichsam eine nur teilweise Befüllung (die jeweilige Messkammer ist in diesem Fall in Bezug auf ihr Volumen nicht vollständig mit flüssigem Messkammermedium gefüllt).

Das Analysesystem umfasst ferner eine Messanordnung. Die Messanordnung ist dazu ausgebildet, im Wege einer photometrischen Messung die in der ersten Messkammer vorliegenden Anionen und/oder Anionen-Erkennungsstoff-Assoziate, und/oder die in der zweiten Messkammer vorliegenden Kationen und/oder Kationen-Erkennungsstoff-Assoziate photometrisch zu messen. Dies kann im Sinne der folgenden drei Alternativen verstanden werden.

In einer ersten Alternative können mit der Messanordnung im Wege einer photometrischen Messung die in der ersten Messkammer vorliegenden Anionen und/oder Anionen-Erkennungsstoff-Assoziate photometrisch gemessen werden. Es können also Anionen und Anionen-Erkennungsstoff-Assoziate photometrisch gemessen werden. Alternativ dazu können nur Anionen, oder nur Anionen-Erkennungsstoff-Assoziate photometrisch gemessen werden. Auf Basis der jeweiligen photometrischen Messung(en) werden dann die Anionen qualitativ und/oder quantitativ bestimmt. Dies kann so verstanden werden, dass die Bestimmung der Anionen qualitativ und quantitativ erfolgt. Alternativ dazu kann die Bestimmung der Anionen nur quantitativ, oder nur qualitativ erfolgen.

Alternativ dazu können mit der Messanordnung im Wege einer photometrischen Messung die in der zweiten Messkammer vorliegenden Kationen und/oder Kationen-Erkennungsstoff-Assoziate photometrisch gemessen werden. Dies kann so verstanden werden, dass Kationen und Kationen-Erkennungsstoff-Assoziate photometrisch gemessen werden. Alternativ dazu können nur Kationen, oder nur Kationen-Erkennungsstoff-Assoziate photometrisch gemessen werden. Auf Basis der jeweiligen photometrischen Messung(en) werden dann die Kationen qualitativ und/oder quantitativ bestimmt. Dies kann so verstanden werden, dass die Bestimmung der Kationen qualitativ und quantitativ erfolgt. Alternativ dazu kann die Bestimmung der Kationen nur quantitativ, oder nur qualitativ erfolgen.

Ferner können mit der Messanordnung sowohl die in der ersten Messkammer vorliegenden Anionen und/oder Anionen-Erkennungsstoff-Assoziate, als auch die in der zweiten Messkammer vorliegenden Kationen und/oder Kationen-Erkennungsstoff-Assoziate gemessen werden. Auf Basis der jeweiligen photometrischen Messung(en) werden dann die Anionen und die Kationen jeweils qualitativ und/oder quantitativ bestimmt. Dies kann so verstanden werden, dass die Bestimmung der Anionen und Kationen sowohl qualitativ als auch quantitativ erfolgt. Alternativ dazu kann die Bestimmung der Anionen und Kationen nur quantitativ, oder nur qualitativ erfolgen.

Das Analysesystem ist vorteilhafterweise universell einsetzbar und einstellbar. Dies kann so verstanden werden, dass das Analysesystem so ausgebildet sein kann, dass Anionen und/oder Kationen, quantitativ und/oder qualitativ, in der ersten und/oder zweiten Messkammer gemessen werden können. Das Analysesystem ist zudem vorteilhafterweise relativ einfach aufgebaut und einfach in der Handhabung, und daher auch zur Nutzung durch einen Endverbraucher (Laien) geeignet.

Das Analysesystem ist zudem durch seinen einfachen Aufbau günstig herstellbar und wartbar.

Die Unteransprüche betreffen vorteilhafte Ausgestaltungen und Weiterbildungen der vorliegenden Erfindung. Die in den Unteransprüchen genannten Merkmale können in beliebiger Kombination zur Weiterbildung des erfindungsgemäßen Analysesystems verwendet werden, soweit dies technisch möglich ist. Dies gilt auch dann, wenn derartige Kombinationen nicht ausdrücklich durch entsprechende Rückbezüge in den Ansprüchen verdeutlicht sind. Insbesondere gilt dies auch über die Kategorie-Grenzen der Patentansprüche hinweg, sodass jene im Kontext des Analysesystems beschriebenen Merkmale und/oder Ausgestaltungen auch Merkmale und/oder Ausgestaltungen des ebenfalls mit der Erfindung vorgeschlagenen Analyseverfahrens sein können und umgekehrt.

Nach einer ersten Ausgestaltung der Erfindung kann in der ersten Messkammer eine erste Mischvorrichtung und/oder in der zweiten Messkammer eine zweite Mischvorrichtung angeordnet sein, um das in der ersten respektive

zweiten Messkammer enthaltene erste respektive zweite flüssige Messkammermedium zu durchmischen. Dies kann so verstanden werden, dass, in einer ersten Alternative, in der ersten Messkammer eine erste Mischvorrichtung angeordnet ist, um das in der ersten Messkammer enthaltene erste flüssige Messkammermedium zu durchmischen und in der zweiten Messkammer eine zweite Mischvorrichtung angeordnet ist, um das in der zweiten Messkammer enthaltene zweite flüssige Messkammermedium zu durchmischen. In einer zweiten Alternative ist nur in der ersten Messkammer eine erste Mischvorrichtung angeordnet. In einer dritten Alternative ist nur in der zweiten Messkammer eine zweite Mischvorrichtung angeordnet. Eine Mischvorrichtung kann grundsätzlich jedwede zur Durchmischung der jeweiligen Kammermedien geeignete Vorrichtung sein. Die Mischvorrichtung kann mechanischer Natur sein (z. B. ein Rührwerk, ein Rührfisch samt Magnetrührer), gleichsam aber auch auf einer Durchströmung oder Ultraschalldurchmischung basieren. Jegliche aus dem Stand der Technik bekannten Misch- oder Rührvorrichtungen können als "Mischvorrichtung" im Sinne der hiesigen Erfindung angesehen werden. Eine gute Vermischung kann die Messgenauigkeit verbessern.

Nach einer weiteren Ausgestaltung der Erfindung kann ein pH-Wert des in der ersten Messkammer enthaltenen ersten flüssigen Messkammermediums und/oder des in der zweiten Messkammer enthaltenen zweiten flüssigen Messkammermediums eingestellt werden. Dies kann so verstanden werden, dass sowohl ein pH-Wert des in der ersten Messkammer enthaltenen ersten flüssigen Messkammermediums als auch des in der zweiten Messkammer enthaltenen zweiten flüssigen Messkammermediums eingestellt wird. Alternativ kann nur ein pH-Wert des in der ersten Messkammer enthaltenen ersten flüssigen Messkammermediums, oder nur ein pH-Wert des in der zweiten Messkammer enthaltenen zweiten flüssigen Messkammermediums eingestellt werden.

Die Einstellung des pH-Werts des in der ersten Messkammer enthaltenen ersten flüssigen Messkammermediums und/oder des in der zweiten Messkammer enthaltenen zweiten flüssigen Messkammermediums ist von Bedeutung, da die optional eingesetzten Erkennungsstoffe jeweils bei bestimmten pH-Werten mit den zu untersuchenden Ionen die gewünschten photometrisch detektierbaren Ionen-Erkennungsstoff-Assoziate bilden. So sind beispielsweise bestimmte Erkennungsstoffe nur bei einem alkalischen pH-Wert des Messkammermediums in der Lage, mit den zu untersuchenden Ionen besagte Ionen-Erkennungsstoff-Assoziate zu bilden. Andere Erkennungsstoffe benötigen wiederum einen sauren pH-Wert des Messkammermediums, um mit den zu untersuchenden Ionen besagte Ionen-Erkennungsstoff-Assoziate bilden zu können. Der pH-Wert eines Mediums kann dabei als ein Maß für den sauren oder basischen Charakter des Mediums verstanden werden. Der pH-Wert kann dabei die Gegenzahl des dekadischen Logarithmus der Wasserstoffionenaktivität darstellen (d.h., je höher die Konzentration von Wasserstoffionen in einem Medium, desto niedriger der pH-Wert) und einen Wert zwischen 0 und 14 annehmen. Dabei kann entsprechen: ein $pH < 7$ einem Medium mit saurer Wirkung, ein $pH = 7$ einem neutralen Medium, und ein $pH > 7$ einem alkalischen Medium mit basischer Wirkung.

Die Einstellung des pH-Werts des ersten und zweiten Messkammermediums kann, wenn die Messkammermedien Wasser umfassen, durch eine an den Elektroden ablaufende Wasserelektrolyse vonstattengehen. Im Rahmen einer Wasserelektrolyse kann durch elektrochemische Vorgänge Wasser in Wasserstoff und Sauerstoff umgewandelt werden, wobei je nach Bedingungen in dem Medium Oxonium-Ionen ($H_3O^+$) oder Hydroxid-Ionen ($OH^-$) gebildet oder verbraucht werden. Beispielsweise kann durch das Anlegen einer Spannung zwischen zwei Elektroden aus Wasser an einer negativ geladenen Elektrode Wasserstoff entstehen, an einer positiv geladenen Elektrode wiederum Sauerstoff.

Beispielsweise kann, bei Anlegen einer Spannung zwischen der ersten und zweiten Elektrode, wodurch die erste Elektrode positiv polarisiert und die zweite Elektrode negativ polarisiert wird, im Rahmen einer Wasserelektrolyse ein Gleichstrom von der negativ geladenen zweiten Elektrode zu der positiv geladenen ersten Elektrode fließen. Unter einem Gleichstromfluss kann dabei eine Wanderung von elektrischen Ladungsträgern, wie Elektronen über im Wasser gelöste Ionen, verstanden werden. Durch bei der Wasserelektrolyse auftretende elektrochemische Vorgänge kann dabei Wasser in der zweiten Messkammer, in der die negativ geladene zweite Elektrode angeordnet ist, basisch und elektronenreich (Wasserstoff-gesättigt), und in der ersten Messkammer, in der die positiv geladene erste Elektrode angeordnet ist, sauer und elektronenarm (Sauerstoff-gesättigt) werden.

Beispielsweise kann eine Wasserelektrolyse so durchgeführt werden, dass sich in der zweiten Messkammer, in der die negativ geladene Elektrode angeordnet ist, ein alkalischer pH-Wert einstellt. Durch den eingestellten alkalischen pH-Wert kann dabei die Fähigkeit eines Erkennungsstoffes, photometrisch detektierbare Ionen-Erkennungsstoff-Assoziate zu bilden, begünstigt werden. Beispielsweise kann die Fähigkeit des Metallindikators Calmagit (welcher einen Erkennungsstoff bereitstellen kann), photometrisch detektierbare Kationen-Metallindikator-Komplexe zu bilden, durch das Einstellen eines alkalischen pH-Wertes in dem zweiten flüssigen Messkammermedium, welches als Erkennungsstoff Calmagit enthält, begünstigt werden.

Grundsätzlich kann jede mögliche Wasserelektrolyseanordnung verwendet werden, die dazu ausgebildet ist, eine Wasserelektrolyse durchzuführen, um den pH-Wert des ersten und zweiten flüssigen Messkammermediums einzustellen. Im Folgenden sollen drei Alternativen einer möglichen Wasserelektrolyseanordnung beschrieben werden. Es wird jedoch an dieser Stelle ausdrücklich darauf hingewiesen, dass durch diese exemplarischen Ausführungen keine Beschränkung auf bestimmte Wasserelektrolyseanordnungen vorgenommen wird.

In einer ersten Alternative kann, zur Einstellung des pH-Wertes des in der ersten Messkammer enthaltenen

ersten flüssigen Messkammermediums und des in der zweiten Messkammer enthaltenen zweiten flüssigen Messkammermedium, zwischen der ersten Messkammer und der zweiten Messkammer eine ladungsdurchlässige Membran angeordnet sein, um einen Ladungsdurchtritt zwischen der ersten Messkammer und der zweiten Messkammer zu ermöglichen. Als eine ladungsdurchlässige Membran kann dabei jede Membran verstanden werden, die einen Durchtritt von elektrischen Ladungsträgern wie Elektronen, Oxonium-Ionen ($H_3O^+$) oder Hydroxid-Ionen ($OH^-$) durch die ladungsdurchlässige Membran ermöglicht. Es können grundsätzlich alle bekannten ladungsdurchlässigen Membranen eingesetzt werden.

In einer zweiten Alternative ist zwischen der ersten Messkammer und einer mit einer ersten Wasserelektrolyselektrode beaufschlagten ersten Wasserelektrolysekammer eine erste ladungsdurchlässige Membran angeordnet, um einen Ladungsdurchtritt zwischen der ersten Messkammer und der ersten Wasserelektrolysekammer zu ermöglichen. Eine Wasserelektrolysezelle wird in diesem Falle gebildet aus der ersten Messkammer sowie der ersten Wasserelektrolysekammer. Dabei bilden die erste Wasserelektrolyseelektrode und die erste Elektrode ein notwendiges Wasserelektrolysezellenelektrodenpaar. Die erste Elektrode bildet dabei neben der ersten Wasserelektrolyseelektrode eine weitere Wasserelektrolyseelektrode dieser Wasserelektrolysezelle. Bei diesem Beispiel ist die erste Wasserelektrolysekammer und die erste Messzelle vorzugsweise mit Wasser befüllt.

Alternativ oder zusätzlich kann eine zweite ladungsdurchlässige Membran zwischen der zweiten Messkammer und einer mit einer zweiten Wasserelektrolyselektrode beaufschlagten zweiten Wasserelektrolysekammer angeordnet sein, um einen Ladungsdurchtritt zwischen der zweiten Messkammer und der zweiten Wasserlektrolysekammer zu ermöglichen. Eine Wasserelektrolysezelle wird in diesem Falle gebildet aus der zweiten Messkammer sowie der zweiten Wasserelektrolysekammer. Dabei bilden die zweite Wasserelektrolyseelektrode und die zweite Elektrode ein notwendiges Wasserelektrolysezellenelektrodenpaar. Die zweite Elektrode bildet dabei neben der zweiten Wasserelektrolyseelektrode eine weitere Wasserelektrolyseelektrode dieser Wasserelektrolysezelle. Bei diesem Beispiel ist die zweite Wasserelektrolysekammer und die zweite Messzelle vorzugsweise mit Wasser befüllt. Zum Funktionsprinzip der Wasserelektrolyse sei auf die vorhergehenden Ausführungen verwiesen.

Durch Einstellen des pH-Wertes auf Basis des Prinzips der Wasserelektrolyse ist keine elektrochemische Vorverstärkung (Anlegen einer Spannung an die erste und zweite Elektrode, samt daraus resultierender Ionenwanderung und sodann gegebenenfalls folgender Bildung von Ionen-Erkennungsstoff-Assoziaten) nötig, um den pH-Wert in jenen Bereich zu bringen, in dem die Erkennungsstoffe eine Bildung von Ionen-Erkennungsstoff-Assoziaten und sodann eine photometrische Erkennung derselben ermöglichen. Dies ist insbesondere für hohe Ionenkonzentrationen in dem flüssigen ionenhaltigen Medium vorteilhaft. Bei geringen Ionenkonzentrationen ist indes eine elektrochemische Vorverstärkung vorteilhaft, um die jeweiligen Ionenkonzentrationen in den Messkammern bzw. an den in den Messkammern angeordneten Elektroden zu erhöhen, um eine aussagekräftige photometrische Messung zu ermöglichen.

In einer dritten Alternative kann die Messzelle zur Einstellung des pH-Wertes in der ersten und zweiten Messkammer eine pH-Einstellkammer aufweisen, wobei zwischen der ersten Messkammer und der pH-Einstellkammer eine erste ladungsdurchlässige Membran angeordnet ist, und wobei zwischen der zweiten Messkammer und der pH-Einstellkammer eine zweite ladungsdurchlässige Membran angeordnet ist. Auch in diesem Kontext kann unter einer ladungsdurchlässigen Membran jede Membran verstanden werden, die einen Durchtritt von elektrischen Ladungsträgern wie Elektronen, Oxonium-Ionen ($H_3O^+$) oder Hydroxid-Ionen ($OH^-$) durch die ladungsdurchlässige Membran ermöglicht. Über diese Kammeranordnung und unter Einsatz der ersten und zweiten Elektrode kann - bei Anlegen einer Spannung an diese Elektroden - der pH-Wert durch Ausführung einer Wasserelektrolyse eingestellt werden. Bei diesem Beispiel bilden die erste und zweite Elektrode ein zur Ausführung der Wasserelektrolyse notwendiges Wasserelektrolyseelektrodenpaar. Zum Funktionsprinzip der Wasserelektrolyse sei auf die vorhergehenden Ausführungen verwiesen.

Ein solcher Aufbau kann sich - im Gegensatz zur Wasserelektrolyse - auch eines anderweitigen pH-Einstellverfahrens bedienen, welches sich die erste und zweite Elektrode zunutze macht. Dabei kann anstelle von Wasser auch ein anderweitiges Medium in der pH-Einstellkammer vorliegen. Die erste und zweite ladungsdurchlässige Membran kann bei diesem Beispiel eine anionenselektive und kationenselektive Membran sein.

Nach einer weiteren Ausgestaltung der Erfindung kann die Messanordnung eine Lichtquelle, einen Messraum, einen zwischen der Lichtquelle und dem Messraum angeordneten ersten Lichtwellenleiter, einen zwischen dem Messraum und einem Detektor angeordneten zweiten Lichtwellenleiter und eine Auswerteeinheit umfassen.

Dabei kann die Lichtquelle dazu ausgebildet sein, einen oder mehrere Lichtstrahl(en) zu erzeugen. Beispielsweise kann die Lichtquelle dazu ausgebildet sein, einen oder mehrere Lichtstrahl(en) einer bestimmten Wellenlänge zu erzeugen. Beispielsweise kann die Lichtquelle eine LED-Lampe, eine Deuterium-Lampe (UV, ultraviolettes Licht), eine Xenon-Blitzlampe (UV, VIS) oder eine Wolfram-HalogenLampe (VIS, sichtbares Licht) sein.

Der Messraum kann durch die erste Messkammer und/oder die zweite Messkammer ausgebildet sein. Dies kann so verstanden werden, dass der Messraum durch die erste Messkammer und die zweite Messkammer, oder nur durch die erste Messkammer, oder nur durch die zweite Messkammer ausgebildet ist. Sofern die erste und/oder zweite Messkammer den Messraum ausbildet, so erfolgt die Messung unmittelbar in der jeweiligen Messkammer, d.h. der seitens der Lichtquelle erzeugte Lichtstrahl tritt (mit einem transmittierten Anteil) zur Messung durch die jeweilige

Messkammer hindurch und wird im Anschluss daran detektiert.

Alternativ kann der Messraum extern zur Messzelle angeordnet sein. Dabei kann der Messraum mit dem ersten und/oder zweiten flüssigen Messkammermedium befüllt sein oder befüllt werden. Dies kann so verstanden werden, dass der Messraum mit dem ersten und zweiten flüssigen Messkammermedium befüllt ist. Alternativ kann der Messraum nur mit dem ersten flüssigen Messkammermedium, oder nur mit dem zweiten flüssigen Messkammermedium befüllt sein. Bei externer Anordnung des Messraums zur Messzelle kann vorgesehen sein, dass das erste respektive zweite flüssige Messkammermedium zum Messraum befördert wird, beispielsweise durch ein flüssigkeitsleitendes Bauteil. Es können auch mehrere externe Messräume vorgesehen sein. Ein externer Messraum kann beispielsweise eine Küvette sein.

Der zwischen der Lichtquelle und dem Messraum angeordnete erste Lichtwellenleiter kann dazu eingerichtet sein, den Lichtstrahl von der Lichtquelle zu dem Messraum weiterzuleiten, und der zwischen dem Messraum und einem Detektor angeordnete zweiten Lichtwellenleiter kann dazu eingerichtet sein, den Lichtstrahl von dem Messraum zu dem Detektor weiterzuleiten.

Es ist zudem vorstellbar, den von der Lichtquelle erzeugten Lichtstrahl vor Eintritt in die erste und zweite Messkammer in Teilstrahlen aufzuteilen, beispielsweise mittels eines Strahlteilers. Alternativ können auch mehrere Lichtquellen zur Erzeugung mehrerer Lichtstrahlen vorgesehen sein, die sodann in die jeweiligen Messräume geleitet werden können. In beiden Fällen kann vorgesehen sein, dass entweder ein bestimmter Teilstrahl oder ein von einer bestimmten Lichtquelle erzeugter Lichtstrahl zur Ausführung einer Nullmessung oder Kalibriermessung in einer der Messkammern (bzw. eine Messraum oder den Messräumen) eingesetzt wird.

Der Detektor kann dazu ausgebildet sein, einen transmittierten Anteil des Lichtstrahls nach Durchgang des Lichtstrahls durch im Messraum befindliches erstes und/oder zweites flüssiges Messkammermedium zu detektieren.

Die Konzentration eines sich in Lösung befindlichen lichtabsorbierenden Stoffes kann über die logarithmierte Intensität an Licht, die eine Lösung (z. B. ein flüssiges Messkammermedium) bei einer bestimmten Wellenlänge absorbiert, die Absorbanz, bestimmt werden. Den Zusammenhang zwischen der Absorbanz $A_2$ bei der Wellenlänge $\lambda$ und der Konzentration c stellt das Lambert-Beer-Gesetz dar:

$$A_\lambda = \varepsilon_\lambda * c * d \text{ (Gleichung 1)}$$

**Hierbei** ist der Extinktionskoeffizient $\varepsilon_\lambda$ eine wellenlängen- und stoffspezifische Konstante und d die Strecke, welche das Licht durch die Probe (z. B. den Messraum) zurücklegt.

Die Auswerteeinheit kann dazu ausgebildet sein, auf Basis eines von dem Detektor erhaltenen Signals, die in dem ionenhaltigen flüssigen Medium vorliegenden Kationen und/oder Anionen qualitativ und/oder quantitativ zu bestimmen. Dies kann so verstanden werden, dass die Auswerteeinheit dazu ausgebildet ist, sowohl Anionen als auch Kationen, oder nur Anionen, oder nur Kationen zu bestimmen, wobei die Bestimmung jeweils sowohl qualitativ als auch quantitativ, oder nur qualitativ, oder nur quantitativ erfolgen kann. Dabei können zunächst Anionen und/oder Kationen in den jeweiligen Messkammermedien quantitativ und/oder qualitativ bestimmt werden. Sodann kann auf dieser Basis qualitativ und/oder quantitativ auf Anionen und/oder Kationen in dem ionenhaltigen flüssigen Medium rückgeschlossen werden. Die Auswerteeinheit kann als Recheneinheit oder Computer zu verstehen sein oder Bestandteil einer Recheneinheit oder eines Computers sein. Eine Recheneinheit oder Computer kann stationär oder portabel sein. Die Auswerteeinheit kann extern zum Detektor angeordnet sein, wobei in diesem Fall der Detektor und die Auswerteeinheit über geeignete Signalkommunikationsschnittstellen verbunden sind. Die Messanordnung (und somit auch die Auswerteeinheit) wird vorzugsweise rechnergestützt betrieben, z. B. unter Einsatz der erwähnten Recheneinheit oder des erwähnten Computers. Die Messanordnung (und somit auch die Auswerteeinheit) kann zum Betrieb auf eine Software, eine Routine, einen Algorithmus oder dergleichen zurückgreifen, die auf der Recheneinheit oder dem Computer ausführbar sind. Die Messanordnung, insbesondere aber die Auswerteeinheit, kann auf Basis von künstlicher Intelligenz (KI), beispielsweise KI-Algorithmen oder entsprechenden (trainierbaren) neuronalen Netzwerken betrieben werden. Die erwähnte Recheneinheit oder der erwähnte Computer können jeglichen notwendigen Schnittstellen, Energieversorgungs- und Speichereinheiten, Datenspeicher etc. enthalten, die zum Betrieb der Messanordnung erforderlich sind. Die erwähnte Recheneinheit oder der erwähnte Computer kann zudem eine Steuer- und Regeleinheit umfassen, mit welcher weitere Komponenten des Analysesystems steuer- und regelbar sind.

Nach einer weiteren Ausgestaltung der Erfindung sind die Lichtquelle, der Detektor und die Auswerteeinheit (also die Messanordnung) extern zur Messzelle angeordnet. Alternativ dazu sind die Lichtquelle, der Detektor und die Auswerteeinheit (also die Messanordnung) in einer gemeinsamen Baueinheit (z. B. einem gemeinsamen Gehäuse) zusammenfasst. Bei einer externen Anordnung kann der Detektor, die Auswerteeinheit und die Lichtquelle ebenfalls in einem gemeinsamen Gehäuse angeordnet sein. Gegebenenfalls können alle Komponenten von einer gemeinsamen Recheneinheit betrieben werden. Vorzugsweise ist das Gehäuse aus einem gewichtssparenden Material, z. B. Kunststoff, gebildet.

Nach einer weiteren Ausgestaltung der Erfindung weist die Probenkammer einen Einlass zur Aufnahme des

ionenhaltigen flüssigen Mediums auf. Ein Einlass kann eine oder mehrere Öffnungen aufweisen, über welche ein Einfüllen oder Einströmen des ionenhaltigen flüssigen Mediums ermöglicht wird. Auch anderweitige Flüssigkeiten, z. B. Spülflüssigkeiten, können über einen solchen Einlass in die Probenkammer gelangen. Die eine oder mehrere Öffnung kann/können verschließbar sein (mit geeigneten Schließmitteln). Der Einlass kann eine Dichtung umfassen. Analog gilt dies für einen Auslass der Probenkammer, welcher ebenfalls eine oder mehrere Öffnungen umfassen kann. Die eine oder mehrere Öffnung des Auslasses kann ebenfalls verschließbar sein. Über den Auslass kann ionenhaltiges flüssiges Medium aus der Probenkammer und damit aus der Messzelle ausgelassen werden.

Dabei kann die Probenkammer entweder durch den Einlass mit dem ionenhaltigen flüssigen Medium befüllt werden, oder von dem ionenhaltigen flüssigen Medium über den Einlass in Richtung eines Auslasses durchströmt werden. Wird die Probenkammer von dem ionenhaltigen flüssigen Medium über den Einlass in Richtung eines Auslasses durchströmt, kann das Analysesystem ferner ein Probenreservoir für das ionenhaltige flüssige Medium umfassen, das über ein flüssigkeitsleitendes Bauteil mit dem Einlass der Probenkammer verbunden ist, und eine Pumpeinheit, die dazu ausgebildet ist, das ionenhaltige flüssige Medium aus dem Probenreservoir über das flüssigkeitsleitende Bauteil durch die Probenkammer zu pumpen.

Nach einer weiteren Ausgestaltung der Erfindung kann das Analysesystem eine zwischen der Pumpeinheit und dem Einlass angeordnete Filtereinheit umfassen, um das ionenhaltige flüssige Medium zu filtern. Dabei kann das ionenhaltige flüssige Medium durch einen in der Filtereinheit angeordneten Filter geleitet werden, wodurch mebranschädigenden Substanzen an dem Filter verbleiben und somit aus dem ionenhaltige flüssige Medium entfernt werden können. Die Anordnung einer Filtereinheit ist besonders vorteilhaft für die Stabilität und Selektivität von Membranen, beispielsweise kationen- oder anionenselektive Membranen, welche insbesondere bei Dauerbelastung durch die membranschädigenden Substanzen zersetzt oder blockiert werden können. Im einfachsten Fall kann unter einer Filtereinheit ein Sieb zu verstehen sein. Ferner können geeignete Filtermaterialien verwendet werden, solange damit die genannten membranschädigenden Substanzen gefiltert werden können (z. B. Filterpapier, Molsieb, Silika, Aktivkohle, Filterkeramiken, Nanoporenfilter etc.).

Nach einer weiteren Ausgestaltung der Erfindung können die in den Messkammern angeordneten Elektroden aus Metall gefertigt sein. Dabei kann das Metall, aus dem die erste in der ersten Messkammer angeordnete Elektrode gefertigt ist, dasselbe Metall sein, aus dem die zweite Elektrode in der zweiten Messkammer gefertigt ist. Alternativ können die in der ersten Messkammer angeordnete erste Elektrode und die in der zweiten Messkammer angeordnete zweite Elektrode aus verschiedenen Metallen gefertigt sein und unterschiedliche elektrochemische Potentiale aufweisen. Im Rahmen der Erfindung können beispielsweise aus Titan oder Platin gefertigte Elektroden zum Einsatz kommen. Vorzugsweise sind sowohl die erste Elektrode in der ersten Messkammer als auch die zweite Elektrode in der zweiten Messkammer aus Titan oder Platin gefertigt. Besonders bevorzugt ist sowohl die erste Elektrode in der ersten Messkammer als auch die zweite Elektrode in der zweiten Messkammer jeweils als ein Titanblech oder Platinblech ausgebildet.

Wie bereits erwähnt, kann das in der ersten Messkammer befindliche erste flüssige Messkammermedium einen ersten Erkennungsstoff enthalten, um bei Durchtritt von Anionen in die erste Messkammer Anionen-Erkennungsstoff-Assoziate zu bilden, und das zweite in der zweiten Messkammer befindliche flüssige Messkammermedium einen zweiten Erkennungsstoff enthalten, um bei Durchtritt von Kationen in die zweite Messkammer Kationen-Erkennungsstoff-Assoziate zu bilden. Die Erfindung ist dabei nicht auf einen bestimmten Erkennungsstoff zur Bildung von Assoziaten festgelegt, vielmehr können sämtliche bekannten und für die jeweiligen zu bestimmenden Ionen als Assoziatbildner geeigneten Erkennungsstoffe im Rahmen der Erfindung verwendet werden.

In diesem Zusammenhang kann unter einem Erkennungsstoff ein solcher chemischer Stoff zu verstehen sein, der zur Erkennung von Ionen in einem flüssigen Medium geeignet ist. Dabei ist der Erkennungsstoff in der Regel für die Erkennung bestimmter Ionen spezifisch einsetzbar. Anders gesagt, ein bestimmter Erkennungsstoff ist in der Regel zur Erkennung bestimmter Ionen, also bestimmter Kationen oder Anionen, geeignet. Im Rahmen der vorliegenden Erfindung können grundsätzlich alle bekannten Erkennungsstoffe zur Erkennung von Ionen eingesetzt werden.

Im Zusammenhang mit der vorliegenden Erfindung kann die Erkennung der Ionen durch den Erkennungsstoff darauf basieren, dass der Erkennungsstoff mit Ionen in einem flüssigen Medium Ionen-Erkennungsstoff-Assoziate bildet, wodurch sich ein detektierbarer chemischer oder physikalischer Zustand des flüssigen Mediums ändert. Dabei kann die detektierbare chemische oder physikalische Zustandsänderung darin bestehen, dass sich, alternativ oder kumulativ, der pH-Wert, die Farbe, die Temperatur oder die Lichtdurchlässigkeit des flüssigen ionenhaltigen Mediums ändert. Die Detektierbarkeit der chemischen oder physikalischen Zustandsänderung kann hierbei so verstanden werden, dass die Änderung des chemischen oder physikalischen Zustands mithilfe einer Messung, wie einer photometrischen Messung, detektiert werden kann.

Im Zusammenhang mit der vorliegenden Erfindung können die Erkennungsstoffe beispielsweise Komplex-Indikatoren bzw. Metallindikatoren, und die Ionen-Erkennungsstoff-Assoziate dementsprechend Metallkationen-Metallindikator-Komplexe sein. In diesem Zusammenhang können Metallindikatoren als solche Erkennungsstoffe verstanden werden, welche mit Metallionen reversible Komplexe bilden. Reversible Komplexe können dabei als solche chemischen Verbindungen verstanden werden, welche aus einem Koordinationszentrum beziehungsweise Zentralion wie einem

Metallion, und Liganden, beispielsweise Chelatliganden, bestehen. Chelatliganden können dabei als solche Liganden verstanden werden, welche mit mehreren Koordinationsstellen an ein Zentralion binden, um einen Chelatkomplex zu bilden. Die chemische Bindung zwischen dem Koordinationszentrum und dem Liganden kann dabei als eine koordinative chemische Bindung verstanden werden, welche von dem Liganden ausgeht, der über freie Elektronenpaare Elektronendichte an das Koordinationszentrum abgibt. Da die koordinative chemische Bindung deutlich schwächer als eine kovalente chemische Bindung ist, ist die Bildung des Komplexes somit ein reversibler Prozess, in dem der reversible Komplex sowie die ungebundenen Liganden und das ungebundene Zentralion in einem dynamischen Gleichgewicht vorliegen. In diesem Zusammenhang stellen Metallindikatoren eine besondere geeignete Form der Chelatkomplexbildner dar, wobei der aus Metallion und Metallindikator gebildete reversible Metall-Indikator-Komplex eine andere detektierbare Farbe aufweist als der reine, ungebundene Metallindikator. Bedingt durch ihre Struktur stellen Metallindikatoren solche Erkennungsstoffe dar, welche sowohl die Farbe als auch den pH-Wert eines flüssigen Mediums, welches zu untersuchende Metallionen und den Metallindikator beziehungsweise daraus gebildete Metall-Indikator-Komplexe enthält, in einer detektierbaren Weise ändern können. Der Metallindikator kann beispielsweise ein Farbstoff wie ein Azofarbstoff sein. Der Metallindikator kann beispielsweise unter dem Handelsnamen Calmagit erwerbliche 2-Hydroxy-5-methyl-benzolazo-1-(2-naphthol-4-sulfonsäure) darstellen (CAS-Nummer 3147-14-6, $C_{17}H_{14}N_2O_5S$) sein.

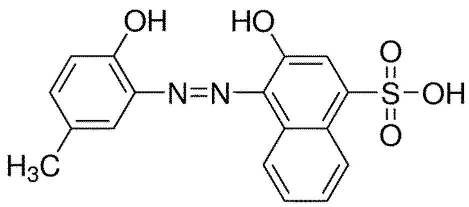

Strukturformel von Calmagit.

Calmagit stellt insbesondere durch seine hohe chemische Stabilität auch in wässriger Lösung einen besonders geeigneten Erkennungsstoff dar. Calmagit liegt in einem flüssigen ionenhaltigen Medium wie einer wässrigen Lösung, je nach pH-Wert des Mediums, in einer einfach bis dreifach deprotonierten Form vor. Als Metallindikator fungiert dabei die zweifach deprotonierte Form. Calmagit kann beispielsweise zur Bestimmung von zweiwertigen Kationen wie Calciumkationen oder Magnesiumkationen oder dreiwertigen Kationen wie Aluminiumkationen eingesetzt werden.

Im Zusammenhang mit der vorliegenden Erfindung können die Erkennungsstoffe ferner Fällungsmittel, und die Ionen-Erkennungsstoff-Assoziate dementsprechend Ionen-Fällungsmittel-Assoziate sein. Unter einem Fällungsmittel kann dabei eine chemische Verbindung verstanden werden, die mit in einem flüssigen Medium vorliegenden Ionen Ionen-Fällungsmittel-Assoziate bildet, welche in besagtem Medium unlöslich sind und daher aus dem Medium ausfallen. Unter der Fällung eines Ions mit einem Fällungsmittel aus einem flüssigen Medium, wie einer wässrigen Lösung, kann dabei das Ausscheiden eines zuvor gelösten Ions in Form von einem Niederschlag bestehend aus Ionen-Fällungsmittel-Assoziaten verstanden werden. Im Zusammenhang mit der vorliegenden Erfindung können alle bekannten Fällungsmittel eingesetzt werden.

Durch die Bildung von in dem flüssigen Medium unlöslichen Ionen-Fällungsmittel-Assoziate kann die Lichtdurchlässigkeit des Mediums in einer detektierbaren Weise reduziert werden. Das Fällungsmittel kann beispielsweise unter dem Handelsnamen Kalignost erwerbliches Natriumtetraphenylborat ($Na[B(C_6H_5)_4]$, CAS-Nummer 143-66-8) sein. Kalignost ist insbesondere zur Erkennung von Kalium-, Ammonium-, Rubidium-, Caesium- und Thalliumionen geeignet.

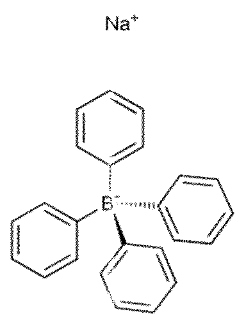

Strukturformel von Kalignost.

Nach einer Ausgestaltung der Erfindung kann der erste Erkennungsstoff und/oder der zweite Erkennungsstoff

aus der Gruppe Calmagit, Dimethylglyoxim, Calconcarbonsäure, Xylenolorange, Eriochromschwarz T, Eriochromblauschwarz R, Ethylendiamintetraacetat (EDTA), Oxalsäure, Kalignost, Murexid, Methylthymolblau, Metallphthalein, Brenzcatechinviolett, 1-(2-Pyridylazo)-2-naphthol, 4-(2-Py-ridylazo)resorcin, Eisen(III)chlorid, oder Mischungen davon, ausgewählt sein. Dies kann so verstanden werden, dass sowohl der erste als auch der erste Erkennungsstoff, oder lediglich der erste Erkennungsstoff, oder lediglich der zweite Erkennungsstoff, aus der Gruppe Calmagit, Dimethylglyoxim, Calconcarbonsäure, Xylenolorange, Eriochromschwarz T, Eriochromblauschwarz R, Ethylendiamintetraacetat (EDTA), Oxalsäure, Kalignost, Murexid, Methylthymolblau, Metallphthalein, Brenzcatechinviolett, 1-(2-Pyridylazo)-2-naphthol, 4-(2-Pyridylazo)resorcin, Eisen(III)chlorid, oder Mischungen davon, ausgewählt ist. Bevorzugt ist sowohl der erste Erkennungsstoff als auch der zweite Erkennungsstoff aus der Gruppe Calmagit, Dimethylglyoxim, Calconcarbonsäure, Xylenolorange, Eriochromschwarz T, Eriochromblauschwarz R, Ethylendiamintetraacetat (EDTA), Oxalsäure, Kalignost, Murexid, Methylthymolblau, Metallphthalein, Brenzcatechinviolett, 1-(2-Py-ridylazo)-2-naphthol, 4-(2-Pyridylazo)resorcin, Eisen(III)chlorid, oder Mischungen davon, ausgewählt. Besonders bevorzugt ist der zweite Erkennungsstoff Calmagit.

Das erfindungsgemäße Analyseverfahren kann, ausgeführt mit dem erfindungsgemäßen Analysesystem, durch den Einsatz verschiedener Erkennungsstoffe ohne großen Aufwand an die Bedingungen verschiedener Einsatzgebiete angepasst werden, ohne dass hierfür technische Änderungen an dem Analysesystem von Nöten sind. Durch die Wahl eines geeigneten Erkennungsstoffes, beispielsweise eines geeigneten Metallindikators, ist zudem eine Unempfindlichkeit der Analyse gegenüber Störionen gegeben.

Im Rahmen der Erfindung können alle bekannten Ionen, die in dem ionenhaltigen flüssigen Medium enthalten sein können, bestimmt werden. Ionen können dabei als elektrisch geladene Teilchen verstanden werden, die aus ungeladenen Atomen oder Molekülen durch die Abgabe oder Anlagerung von Elektronen entstehen. Anionen und Kationen können ein- oder mehrwertig geladen sein.

Kationen können dabei grundsätzlich als positiv geladene Ionen verstanden werden, welche aus ungeladenen Atomen oder Molekülen durch die Abgabe von Elektronen entstehen. Anionen wiederum können als negativ geladene Ionen verstanden werden, welche aus ungeladenen Atomen oder Molekülen durch die Anlagerung von Elektronen entstehen. Bei Anlegen einer Spannung zwischen zwei Elektroden, wodurch eine Elektrode negativ polarisiert und die andere positiv polarisiert wird, werden in einem flüssigen Medium gelöste Kationen aufgrund ihrer positiven Ladung zur negativ geladenen Elektrode, Anionen wiederum aufgrund ihrer negativen Ladung zur positiv geladenen Elektrode befördert.

Nach einer Ausgestaltung der Erfindung können in dem ionenhaltigen flüssigen Medium vorliegende Kationen wie Eisen(II)-Ionen, Eisen(III)-Ionen, Kupfer(II)-Ionen, Magnesium(II)-Ionen, Calcium(II)-Ionen, Mangan(II)-Ionen, Kalium(I)-Ionen, Ammoniumkationen ($NH_4^+$), oder Mischungen daraus, bestimmt werden. Alternativ oder kumulativ dazu können in dem ionenhaltigen flüssigen Medium vorliegende Anionen wie Nitrat-Ionen ($NO_3^-$), Nitrit-Ionen ($NO_2^-$), Phosphat-Ionen ($PO_4^{3-}$), oder Mischungen daraus sein. Bevorzugt sind die in dem ionenhaltigen flüssigen Medium vorliegenden Kationen, die bestimmt werden, Eisen(II)-Ionen, Eisen(III)-Ionen, Kupfer(II)-Ionen, Magnesium(II)-Ionen, Calcium(II)-Ionen, Mangan(II)-Ionen, Kalium(I)-Ionen, Ammoniumkationen ($NH_4^+$), oder Mischungen daraus, und die in dem ionenhaltigen flüssigen Medium vorliegenden Anionen, die bestimmt werden, Nitrat-Ionen ($NO_3^-$), Nitrit-Ionen ($NO_2^-$), Phosphat-Ionen (**$PO_4^{3-}$** ), oder Mischungen daraus.

Zudem kann das erste und/oder zweite flüssige Messkammermedium einen weiteren Komplexbildner umfassen. Unter einem weiteren Komplexbildner kann dabei jeder chemische Stoff verstanden werden, der in der Lage ist, in den Messkammermedien enthaltene störende Stoffe zu maskieren. Unter einer Maskierung störender Stoffe kann dabei eine Komplexierung der störenden Stoffe durch den weiteren Komplexbildner verstanden werden, wodurch die störenden Stoffe zu maskierten Stoffen umgesetzt werden. Unter maskierten Stoffen können dabei Stoffe verstanden werden, welche nicht mehr in der Lage sind, das erfindungsgemäße Analyseverfahren, insbesondere photometrische Messungen in den Messkammern, zu stören. Dabei kann der weitere Komplexbildner, analog einer hierin beschriebenen Regeneration von Erkennungsstoffen, regeneriert werden.

Nach einer weiteren Ausgestaltung der Erfindung kann das erste und/oder zweite flüssige Messkammermedium zur Erhöhung der Leitfähigkeit einen Elektrolyten umfassen. Im Zusammenhang mit der vorliegenden Erfindung kann ein Elektrolyt als ein zum ersten und/oder zweiten flüssigen Messkammermedium zugegebener chemischer Stoff verstanden werden, der dazu geeignet ist, die Leitfähigkeit des jeweiligen Messkammermediums zu erhöhen. In diesem Zusammenhang kann der Elektrolyt bei Zugabe zu einem Messkammermedium in seine Ionen dissoziieren und beim Anlegen einer Spannung zwischen in den Messkammern angeordneten Elektroden unter dem Einfluss des dabei entstehenden elektrischen Feldes den elektrischen Strom leiten, wodurch die Leitfähigkeit des Messkammermediums im Vergleich zu einem Messkammermedium, das keinen Elektrolyten enthält, erhöht wird. Als Leitfähigkeit wird dabei die Fähigkeit bezeichnet, elektrischen Strom zu leiten. Grundsätzlich können alle bekannten Elektrolyten eingesetzt werden. Besonders bevorzugt ist der Elektrolyt Natriumsulfat.

Alternativ kann zur Erhöhung der Leitfähigkeit des flüssigen Mediums ein Ionenaustauschermaterial in der Probenkammer angeordnet sein. In diesem Zusammenhang kann das flüssige Medium das in der Probenkammer

angeordnete Ionenaustauschermaterial durchströmen, wobei Ionen einer Ionenart am Ionenaustauschermaterial gebunden und eine äquivalente Ladungsmenge vorher an das Ionenaustauschermaterial gebundener Ionen einer anderen Ionenart in das Medium abgegeben werden, wodurch die Leitfähigkeit des flüssigen Mediums erhöht wird. Dabei kann ein Ionenaustauschermaterial als ein solches Material verstanden werden, das eine ursprünglich im flüssigen Medium vorliegende eine Ionenart gegen eine ursprünglich an den Ionenaustauscher gebundene andere Ionenart austauschen kann. Das Ionenaustauschermaterial kann beispielsweise Aluminiumoxid, Chlorophyll, Colestyramin, Colesevelam, ein Kunstharz basierend auf mindestens einem Polymermaterial, oder ein Zeolith wie Zeolith A (Sasil) sein.

Dabei kann das Ionenaustauschermaterial ein Kationenaustauschermaterial, ein Anionenaustauschermaterial, oder ein amphoteres Ionenaustauschermaterial sein. In diesem Zusammenhang kann ein Kationenaustauschermaterial eine Kationenart, die im flüssigen ionenhaltigen Medium gelöst ist, binden und eine ursprünglich an das Ionenaustauschermaterial gebundene andere Kationenart in das Medium freisetzen, wodurch im Laufe des Austauschprozesses die eine gegen die andere Kationenart ausgetauscht wird. Ein Anionenaustauschermaterial kann dementsprechend Anionen einer Anionenart gegen Anionen einer anderen Anionenart austauschen. Ein amphoteres Ionenaustauschermaterial kann wiederum gleichzeitig Anionen und Kationen austauschen.

Dabei enthalten Kationenaustauschermaterialien an ihrer Oberfläche anionische funktionelle Gruppe, wie beispielsweise Carbonsäure- oder Sulfonsäuregruppen, mit abdissoziierbaren Kationen. Anionenaustauscher enthalten wiederum kationische funktionelle Gruppen, wie beispielsweise quartäre Ammoniumgruppen, die ihr Gegenanion austauschen können.

Nach einer weiteren Ausgestaltung der Erfindung kann die anionenselektive Membran und/oder die kationenselektive Membran jeweils aus einem Membranmaterial gefertigt sein, das ein erstes und/oder ein zweite Polymermaterial umfasst. Das erste Polymermaterial enthält dabei an einer Polymermaterialoberfläche angeordnete funktionelle Gruppen, die eine Anionenselektivität der anionenselektiven Membran und/oder eine Kationenselektivität der kationenselektiven Membran bereitstellen. Grundsätzlich sind dabei solche funktionellen Gruppen zur Bereitstellung einer Anionenselektivität einer anionenselektiven Membran geeignet, welche positiv polarisierbar sind, wodurch Anionen der Durchgang durch die Membran ermöglicht wird, jedoch Kationen abgestoßen und damit am Durchtritt durch die Membran gehindert werden. Entsprechend sind solche funktionellen Gruppen zur Bereitstellung einer Kationenselektivität einer kationenselektiven Membran geeignet, welche negativ polarisierbar sind, wodurch Kationen der Durchgang durch die Membran ermöglicht wird, jedoch Anionen abgestoßen und damit am Durchtritt durch die Membran gehindert werden.

Vorzugsweise ist das erste Polymermaterial modifiziertes Polystyrol (PS) mit an einer Polymermaterialoberfläche angeordneten funktionellen Gruppen. Dabei sind die an der Polymermaterialoberfläche angeordneten funktionellen Gruppen zur Bereitstellung der Anionenselektivität der anionenselektiven Membran grundsätzlich positiv polarisierbare funktionelle Gruppen, vorzugsweise quaternäre Amine. Alternativ oder kumulativ sind die an der Polymermaterialoberfläche angeordneten funktionellen Gruppen zur Bereitstellung der Kationenselektivität der kationenselektiven Membran negativ polarisierbare funktionelle Gruppen, vorzugsweise Carbonsäure- oder Sulfonsäuregruppen.

Das Membranmaterial kann ferner zur mechanischen Verstärkung der entsprechenden anionenselektiven Membran und/oder der kationenselektiven Membran ein zweites Polymermaterial umfassen. Unter einer mechanischen Verstärkung einer Membran kann dabei eine erhöhte Widerstandsfähigkeit der Membran gegen mechanische Einflüsse, wie beispielsweise einen mechanischen Stoß, verstanden werden, welche durch den Einbau eines zweiten Polymermaterials in die Membran erreicht werden kann. Das zweite Polymermaterial umfasst dabei mindestens ein zur mechanischen Verstärkung geeignetes Polymer. Vorzugsweise ist das zweite Polymermaterial Polyvinylchlorid (PVC), Polypropylen (PP) oder Polyethylenterephthalat (PET).

Nach einer weiteren Ausgestaltung der Erfindung kann vorgesehen sein, dass das Analysesystem eine Mehrzahl von ersten Messkammern und/oder zweiten Messkammern umfasst. Insbesondere können mehrere Paare von ersten und zweiten Messkammern vorgesehen sein. Zwischen den jeweiligen Paaren können Probenkammern angeordnet sein, sodass insgesamt eine Mehrzahl von Messzellen vorliegen kann, die jeweils gebildet sind aus einer ersten Messkammer, einer zweiten Messkammer und einer jeweils dazwischen angeordneten Probenkammer. Mehrere Messzellen können vorteilhaft sein um unterschiedliche Ionenarten qualitativ und/oder quantitativ parallel untersuchen zu können oder unterschiedliche Messbedingungen (z. B. unterschiedliche Erkennungsstoffe etc.) zu gewährleisten.

Nach einer weiteren Ausgestaltung der Erfindung kann das Analysesystem als eine kompakte Sonde mit integrierter Pumpeinheit unmittelbar in einem Probenreservoir angeordnet werden. Unter einem Probenreservoir kann dabei ein statisches oder fließendes zu untersuchendes Medium, beispielsweise ein stehendes oder fließendes Gewässer, wie ein See oder Fluss, oder ein industrielles stehendes oder fließendes Medium, wie beispielsweise ein Durchflussreaktor verstanden werden. Auch Kläranlagen, Wasseraufbereitungsanlagen etc. können stehende oder fließende Medien bereitstellen, die mit dem beschriebenen Analysesystem untersucht werden können. Gleichsam kann auch ein mit Wasser befülltes Aquarium ein Probenreservoir bereitstellen. Das in einem Aquarium enthaltene Wasser kann das statische oder fließende zu untersuchende Medium bilden. Ein Analysesystem in Form einer kompakten Sonde mit integrierter Pumpeinheit hat unter anderem den Vorteil, dass die kompakte Sonde sehr einfach und kostengünstig

herzustellen und zu warten ist, und direkt in einem Probenreservoir platziert werden kann, wodurch sehr präzise Messwerte, auch im Zuge einer Echtzeit-Messung, wie einer Online- oder Inline-Messung, erhalten werden können.

Alternativ kann das Analysesystem auch als eine kompakte Sonde ohne Pumpeinheit ausgebildet sein. Dabei kann das Analysesystem unmittelbar in einem Probenreservoir mit einem Fließmedium (z. B. Fließgewässer) mit einer Fließgeschwindigkeit angeordnet sein. Aufgrund der Fließgeschwindigkeit des Fließmediums kann hierbei die Notwendigkeit entfallen, das ionenhaltige flüssige Medium durch die Probenkammer zu pumpen, sodass die Anordnung einer Pumpeinheit entfallen kann. Ein solches Analysesystem ist aus dem Grunde von Vorteil, dass es einfach und kostengünstig herzustellen, zu verwenden und zu warten ist. Auch in diesem Fall kann das Probenreservoir ein Aquarium sein.

Alternativ kann das Analysesystem extern zu einem Probenreservoir (mit einem zu untersuchenden fließenden oder einem statischen Medium) angeordnet werden. Sodann wird die Probenkammer mit ionenhaltigem flüssigen Medium aus dem Reservoir befüllt. Auch kann ionenhaltiges flüssiges Medium aus einem solchen Reservoir in die Probenkammer geleitet (z. B. gepumpt) werden. Auch in diesem Fall kann das Probenreservoir ein Aquarium sein.

Wie bereits erwähnt, wird die der vorliegenden Erfindung zugrunde liegende Aufgabenstellung ebenso mit einem Analyseverfahren gemäß dem Patentanspruch 13 gelöst. Das genannte Analyseverfahren umfasst zumindest die folgenden Verfahrensschritte S2 und S3:

- S2: photometrisches Messen von Anionen, Anionen-Erkennungsstoff-Assoziaten, Kationen und/oder Kationen-Erkennungsstoff-Assoziaten mit der Messanordnung,
- S3: qualitatives und/oder quantitatives Bestimmen der Anionen und/oder Kationen auf Basis der photometrischen Messung in Schritt S2.

Dabei betrifft der Verfahrensschritt S2

- photometrisches Messen von in der ersten Messkammer befindlichen Anionen und/oder Anionen-Erkennungsstoff-Assoziaten mit der Messanordnung, und/oder
- photometrisches Messen von in der zweiten Messkammer befindlichen Kationen und/oder Kationen-Erkennungsstoff-Assoziaten mit der Messanordnung.

Der Verfahrensschritt S3 betrifft sodann qualitatives und/oder quantitatives Bestimmen der Anionen und/oder Kationen auf Basis der photometrischen Messung in Schritt S2. Schritt S3 wird kann insbesondere mit einer Auswerteeinheit durchgeführt werden, die bereits an mehreren Stellen beschrieben wurde.

Dies kann im Sinne der folgenden drei Alternativen verstanden werden.

In einer ersten Alternative können in Schritt S2 mit der Messanordnung im Wege einer photometrischen Messung die in der ersten Messkammer vorliegenden Anionen und/oder Anionen-Erkennungsstoff-Assoziate photometrisch gemessen werden. Es können also Anionen und Anionen-Erkennungsstoff-Assoziate photometrisch gemessen werden. Alternativ dazu können nur Anionen, oder nur Anionen-Erkennungsstoff-Assoziate photometrisch gemessen werden.

Auf Basis der jeweiligen photometrischen Messung(en) in Schritt S2 werden dann in Schritt S3 die Anionen qualitativ und/oder quantitativ bestimmt. Dies kann so verstanden werden, dass die Bestimmung der Anionen qualitativ und quantitativ erfolgt. Alternativ dazu kann die Bestimmung der Anionen nur quantitativ, oder nur qualitativ erfolgen. Die qualitative und/oder quantitative Bestimmung kann insbesondere mit einer Auswerteeinheit durchgeführt werden, die bereits an mehreren Stellen beschrieben wurde.

Alternativ dazu können in Schritt S2 mit der Messanordnung im Wege einer photometrischen Messung die in der zweiten Messkammer vorliegenden Kationen und/oder Kationen-Erkennungsstoff-Assoziate photometrisch gemessen werden. Dies kann so verstanden werden, dass Kationen und Kationen-Erkennungsstoff-Assoziate photometrisch gemessen werden. Alternativ dazu können nur Kationen, oder nur Kationen-Erkennungsstoff-Assoziate photometrisch gemessen werden. Auf Basis der jeweiligen photometrischen Messung(en) in Schritt S2 werden dann in Schritt S3 die Kationen qualitativ und/oder quantitativ bestimmt. Dies kann so verstanden werden, dass die Bestimmung der Kationen qualitativ und quantitativ erfolgt. Alternativ dazu kann die Bestimmung der Kationen nur quantitativ, oder nur qualitativ erfolgen. Die qualitative und/oder quantitative Bestimmung kann insbesondere mit einer Auswerteeinheit durchgeführt werden, die bereits an mehreren Stellen beschrieben wurde.

Ferner können mit der Messanordnung in Schritt S2 sowohl die in der ersten Messkammer vorliegenden Anionen und/oder Anionen-Erkennungsstoff-Assoziate, als auch die in der zweiten Messkammer vorliegenden Kationen und/oder Kationen-Erkennungsstoff-Assoziate gemessen werden. Auf Basis der jeweiligen photometrischen Messung(en) in Schritt S2 werden dann in Schritt S3 die Anionen und die Kationen jeweils qualitativ und/oder quantitativ bestimmt. Dies kann so verstanden werden, dass die Bestimmung der Anionen und Kationen gleichzeitig qualitativ und quantitativ erfolgt. Alternativ dazu kann die Bestimmung der Anionen und Kationen nur quantitativ, oder nur qualitativ erfolgen. Die qualitative und/oder quantitative Bestimmung kann insbesondere mit einer Auswerteeinheit durchgeführt werden, die bereits an

mehreren Stellen beschrieben wurde.

Eine photometrische Messung kann dabei ein solches Messverfahren darstellen, bei dem mit Hilfe einer Lichtquelle flüssige Medien hinsichtlich ihrer Zusammensetzung, beispielsweise hinsichtlich darin vorliegender Ionen, analysiert werden können. So kann in einer photometrischen Analyse beispielsweise eine chemische Substanz über ihre spezifische Farbreaktion und Lichtabsorption in Abhängigkeit zur chemischen Eigenschaft bei einer bestimmten Wellenlänge analysiert werden. Dabei kann, für eine photometrische Messung, von einer Lichtquelle ein Lichtstrahl erzeugt werden, welcher durch einen Messraum geleitet wird. Der Lichtstrahl kann dann als ein transmittierter Anteil des Lichtstrahls aus dem Messraum wieder hinaustreten und sodann von einem Detektor detektiert werden. Der transmittierte Anteil des Lichtstrahls kann dabei als ein Lichtstrahl mit reduzierter Lichtintensität im Vergleich zu dem in den Messraum eingeleiteten Lichtstrahl verstanden werden. Die Reduzierung der Lichtintensität kann dabei durch Wechselwirkungen, wie Absorption, Streuung, Beugung oder Reflexion, des Lichtstrahls mit dem in dem Messraum befindlichen Medium, welches die zu untersuchenden Ionen enthält, begründet sein. Dabei kann die Transmission (Extinktion, Absorbanz) als ein Maß für die Lichtundurchlässigkeit des zu untersuchenden ionenhaltigen Mediums verstanden werden, die durch folgende Gleichung beschrieben wird:

$$E = \log I_0/I_D = \log 1/D \text{ (Gleichung 2)}$$

wobei $I_0$: Intensität des Lichtstrahl vor Durchgang durch den Messraum,
$I_D$: Intensität des Lichtstrahls nach Durchgang durch den Messraum,
D: Lichtdurchlässigkeit $I_D/I_0$.

Die photometrische Analyse (die mit der beschriebenen Messanordnung vorgenommen werden kann) zeichnet sich durch Schnelligkeit, hohe Selektivität und geringen Zeitbedarf aus. Besondere Bedeutung hat sie zur genauen Erfassung geringer Gehalte von Ionen in einem flüssigen Medium, beispielsweise zur Bestimmung von Metallen im Spurenbereich. Der von der Lichtquelle erzeugte Lichtstrahl basiert dabei vorzugsweise auf ultraviolettem (UV) oder sichtbarem (VIS) Licht. Im Wege einer photometrischen Messung kann nun die Transmission eines flüssigen Mediums in Abhängigkeit von der Wellenlänge des eingestrahlten Lichts gemessen, und daraus ein Transmissionsspektrum erhalten werden. Über das Transmissionsspektrum können sodann die Ionen über charakteristische Erscheinungen in dem Transmissionsspektrum auf qualitative Weise, und/oder mithilfe geeigneter Berechnungsmethoden auch auf quantitative Weise bestimmt werden. Charakteristische Erscheinungen des Transmissionsspektrums können charakteristische Kurvenmerkmale sein, beispielsweise Peaks, Maxima, Minima, Wendepunkte, Steigungen, Glattheit, Krümmung, wellenlängenabhängige Positionen (oder Verschiebungen) der vorgenannten Merkmale oder Abstände von charakteristischen Kurvenmerkmalen im Spektrum. Auch Verhältnisse von charakteristischen Kurvenmerkmalen (z. B. Verhältnisse gemittelter Intensitäten oder dergleichen) können zur Auswertung herangezogen werden. Die qualitative und/oder quantitative Bestimmung kann insbesondere mit einer Auswerteeinheit durchgeführt werden, die bereits an mehreren Stellen beschrieben wurde.

Ein weiterer optionaler Schritt S1 betrifft zudem,

- Bilden von Anionen-Erkennungsstoff-Assoziaten aus in der ersten Messkammer befindlichen Anionen und dem ersten Erkennungsstoff, und/oder
- Bilden von Kationen-Erkennungsstoff-Assoziaten aus in der zweiten Messkammer befindlichen Kationen und dem zweiten Erkennungsstoff.

Die Unteransprüche betreffen vorteilhafte Ausgestaltungen und Weiterbildungen der vorliegenden Erfindung. Die in den Unteransprüchen genannten Merkmale können in beliebiger Kombination zur Weiterbildung des erfindungsgemäßen Analyseverfahrens verwendet werden, soweit dies technisch möglich ist. Dies gilt auch dann, wenn derartige Kombinationen nicht ausdrücklich durch entsprechende Rückbezüge in den Ansprüchen verdeutlicht sind. Insbesondere gilt dies auch über die Kategorie-Grenzen der Patentansprüche hinweg.

Nach einer vorteilhaften Ausgestaltung des erfindungsgemäßen Verfahrens kann, vorgelagert zum optionalen Schritt S1, oder vorgelagert zum Schritt S2, ein Schritt S0-a ausgeführt werden. Dabei betrifft Schritt S0-a ein Anlegen einer Spannung zwischen der ersten Elektrode und der zweiten Elektrode, wodurch die erste Elektrode positiv und die zweite Elektrode negativ polarisiert wird, und wodurch Anionen von der Probenkammer durch die anionenselektive Membran in Richtung der in der ersten Messkammer angeordneten ersten Elektrode und Kationen von der Probenkammer durch die kationenselektive Membran in Richtung der in der zweiten Messzelle angeordneten zweiten Elektrode befördert werden.

Nach einer weiteren vorteilhaften Ausgestaltung des erfindungsgemäßen Analyseverfahrens kann, vorgelagert zum optionalen Schritt S1, oder vorgelagert zum Schritt S2, ein Schritt S0-b ausgeführt werden. Dabei betrifft Schritt S0-b

ein Einstellen eines pH-Wertes des in der ersten Messkammer enthaltenen ersten flüssigen Messkammermediums und/oder des in der zweiten Messkammer enthaltenen zweiten flüssigen Messkammermediums mit einem Analysesystem nach Anspruch 3.

Nach einer weiteren vorteilhaften Ausgestaltung des erfindungsgemäßen Analyseverfahrens kann, nach dem Schritt S3, oder vorgelagert zum Schritt S0-a, ein Schritt S4 ausgeführt werden. Dabei betrifft Schritt S4 ein Anlegen einer im Vergleich zu der in Schritt S0-a angelegten, umgekehrten Spannung zwischen der ersten Elektrode und der zweiten Elektrode, wodurch die erste Elektrode negativ und die zweite Elektrode positiv polarisiert wird.

Dadurch werden,

- die im optionalen Schritt S1 in der ersten Messkammer gebildeten Anionen-Erkennungsstoff-Assoziate zu Anionen und dem ersten Erkennungsstoff zurückgebildet und Anionen von der ersten Messkammer durch die anionenselektive Membran in die Probenkammer zurückbefördert, wodurch der erste Erkennungsstoff regeneriert wird, und/oder
- die im optionalen Schritt S1 in der zweiten Messkammer gebildeten Kationen-Erkennungsstoff-Assoziate zu Kationen und dem zweiten Erkennungsstoff zurückgebildet und Kationen von der zweiten Messkammer durch die kationenselektive Membran in die Probenkammer zurückbefördert, wodurch der zweite Erkennungsstoff regeneriert wird.

Grundsätzlich können sämtliche Verfahrensschritte betreffend ein Messen und/oder Auswerten rechnergestützt erfolgen, d.h. unter Einsatz einer geeigneten Recheneinheit. Eine solche Recheneinheit kann auf Rechenroutinen, Algorithmen, Software etc. zurückgreifen. Eine solche Recheneinheit kann - wie erwähnt - ein Computer sein. Eine Recheneinheit kann programmierbar sein.

Weitere Vorteile, Ausgestaltungen und Weiterbildungen, die im Zusammenhang mit dem erfindungsgemäßen Analysesystem bzw. dem erfindungsgemäßen Analyseverfahren stehen, sind anhand der nachfolgend beschriebenen Ausführungsbeispiele genauer erläutert. Diese sollen dem Fachmann die Erfindung verdeutlichen und ihn in die Lage versetzen, die Erfindung auszuführen, ohne jedoch die Erfindung zu beschränken. Im Zusammenhang der Beschreibung der genannten Ausführungsbeispiele wird auf die folgenden Figuren Bezug genommen, anhand welcher das erfindungsgemäße Analysesystem bzw. das erfindungsgemäße Analyseverfahren genauer erläutert wird. So zeigt

Figur 1 eine schematische Darstellung einer Messzelle, die in einer Ausführungsform des erfindungsgemäßen Analysesystems verwendet wird;

Figur 2 eine schematische Darstellung eines Analysesystem gemäß einer Ausführungsform der Erfindung;

Figur 3 ein beispielhaftes Absorptionsspektrum von Calconcarbonsäure als Beispiel für einen Erkennungsstoff;

Figur 4 ein beispielhaftes Absorptionsspektrum von Calmagit als weiteres Beispiel eines Erkennungsstoffs;

Figur 5 Absorptionsspektren verschiedener Metallindikatoren zusammen mit verschiedenen zu bestimmenden Ionen.

Figur 1 zeigt eine Messzelle 1, die in einem erfindungsgemäßen Analysesystem nach Figur 2 zum Einsatz kommt. Das Analysesystem ist zur qualitativen und/oder quantitativen Bestimmung von Ionen in einem ionenhaltigen flüssigen Medium 9 ausgebildet. Die Ionen können Kationen K oder Anionen A sein. Wie in Figur 1 gezeigt, umfasst die Messzelle eine erste Messkammer 3, eine zweite Messkammer 4, sowie eine zwischen der ersten Messkammer 3 und der zweiten Messkammer 4 angeordnete Probenkammer 2, wobei die Probenkammer 2 zur Aufnahme des ionenhaltigen flüssigen Mediums 9 ausgebildet ist. Die Figur 1 zeigt exemplarisch eine von einem ionenhaltigen flüssigen Medium 9 durchströmte Probenkammer 2. In der ersten Messkammer 3 ist eine erste Elektrode 5 und in der zweiten Messkammer 4 eine zweite Elektrode 6 angeordnet. Zwischen der ersten und zweiten Elektrode 5, 6 ist eine Spannung anlegbar, wodurch die erste Elektrode 5 positiv und die zweite Elektrode 6 negativ polarisierbar ist, und wodurch die Anionen A in Richtung der ersten Elektrode 5 und die Kationen K in Richtung der zweiten Elektrode 6 beförderbar sind. Zwischen der ersten Messkammer 3 und der Probenkammer 2 ist eine anionenselektive Membran 7 sowie zwischen der zweiten Messkammer 4 und der Probenkammer 2 eine kationenselektive Membran 8 angeordnet. Die anionenselektive Membran 7 ist dazu ausgebildet, einen Durchtritt von Anionen A aus der Probenkammer 2 in die erste Messkammer 3 zu ermöglichen. Die kationenselektive Membran 8 ist dazu ausgebildet, einen Durchtritt von Kationen K aus der Probenkammer 2 in die zweite Messkammer 4 zu ermöglichen. Die erste Messkammer 3 ist mit einem ersten flüssigen Messkammermedium befüllt. Das erste flüssige Messkammermedium kann das ionenhaltige flüssige Medium 9 sein. In dem ersten flüssigen Messkammermedium ist optional ein erster Erkennungsstoff E-1 enthalten, um bei Durchtritt der Anionen A in die erste Messkammer 3 Anionen-Erkennungsstoff-Assoziate A-E-1 zu bilden. Die zweite Messkammer 4 ist mit einem zweiten flüssigen Messkammermedium befüllt. In dem zweiten flüssigen Messkammermedium ist optional ein zweiter Erkennungsstoff E-2 enthalten, um bei Durchtritt der Kationen K in die zweite Messkammer 4 Kationen-Erkennungsstoff-Assoziate K-E-2 zu bilden.

Figur 2 zeigt die Messzelle aus Figur 1 in stark schematisierter Form sowie eine Messanordnung. Die Messanordnung ist dazu ausgebildet im Wege einer photometrischen Messung die in der ersten Messkammer 3 vorliegenden Anionen A und/oder Anionen-Erkennungsstoff-Assoziate A-E-1 photometrisch zu messen, und auf Basis dieser Messung(en) die Anionen A qualitativ und/oder quantitativ zu bestimmen. Alternativ oder zusätzlich ist die Messanordnung dazu ausgebildet im Wege einer photometrischen Messung die in der zweiten Messkammer 4 vorliegenden Kationen K und/oder Kationen-Erkennungsstoff-Assoziate K-E-2 photometrisch zu messen, und auf Basis dieser Messung(en) die Kationen K qualitativ und/oder quantitativ zu bestimmen.

Wie figürlich dargestellt umfasst die Messanordnung eine Lichtquelle 10, die dazu ausgebildet ist, einen Lichtstrahl zu erzeugen. Ferner umfasst die Messanordnung einen Messraum, wobei der Messraum gemäß dem vorliegenden Beispiel durch die erste Messkammer 3 und die zweite Messkammer 4 ausgebildet ist.

Zwischen der Lichtquelle 10 und der ersten Messkammer 3 sowie der zweiten Messkammer 4 (diese fungieren als Messräume) ist ein erster Lichtwellenleiter 12a angeordnet, der dazu eingerichtet ist, den Lichtstrahl von der Lichtquelle 10 zu dem Messraum (also der ersten Messkammer 3 und zweiten Messkammer 4) weiterzuleiten. Zwischen der ersten Messkammer 3 und zweiten Messkammer 4 und einem Detektor 13 ist ein zweiter Lichtwellenleiter 12b angeordnet, der dazu eingerichtet ist, den Lichtstrahl von der ersten Messkammer 3 respektiven zweiten Messkammer 4 zu dem Detektor 13 weiterzuleiten, wobei der Detektor 13 dazu ausgebildet ist, einen transmittierten Anteil des Lichtstrahls nach Durchgang des Lichtstrahls durch im Messraum befindliches erstes oder zweites flüssiges Messkammermedium zu detektieren. Ferner ist eine Auswerteeinheit 14 vorgesehen, die dazu ausgebildet ist, auf Basis eines von dem Detektor 13 erhaltenen Signals, die in dem ionenhaltigen flüssigen Medium 9 vorliegenden Kationen und/oder Anionen qualitativ und/oder quantitativ zu bestimmen. Ersichtlich ist die Lichtquelle 10, der Detektor 13 und die Auswerteeinheit 14 in einem externen Teil (z. B. einem Gehäuse 11) zur Messzelle 1 angeordnet. Die Auswerteeinheit 14 wird von einem Anwender 15 bedient, kann von diesem programmiert oder ausgelesen werden.

Figur 2 zeigt ferner, dass aus einem Probenreservoir 18 über ein flüssigkeitsleitendes Bauteil 17 (z. B. eine Leitung, ein Rohr, Schlauch oder dergleichen) ionenhaltiges flüssiges Medium 9 in die Probenkammer 2 geleitet wird. Aus der Probenkammer 2 wird das ionenhaltige flüssige Medium zudem wieder entfernt, beispielsweise unter Einsatz einer Pumpeinheit 16.

Figur 5 zeigt die Lage der Absorptionsmaxima verschiedener Metallkation-Metallindikator-Komplexe nach entsprechender photometrischer Messung. Eine gute Unterscheidbarkeit der Metallkationen ist zu erwarten, wenn die Absorptionsmaxima möglichst weit voneinander entfernt liegen und sich nicht überdecken. Dies ist insbesondere bei Calmagit und Calconcarbonsäure der Fall, weswegen in Figur 3 und 4 entsprechende Absorptionsspektren von alleinig Calcocarbonsäure (Fig. 3) und alleinig Calmagit (Fig. 4) im Vergleich zu den Absorptionsspektren von Calcocarbonsäure-Ionen-Assoziaten (mit den Ionen $Cu^{2+}$, $Ca^{2+}$, $Fe^{3+}$, $Mg^{2+}$ und $Fe^{2+}$) in Figur 3 bzw. Calmagit-Ionen-Assoziaten (mit den Ionen $Cu^{2+}$, $Ca^{2+}$, $Fe^{3+}$, $Mg^{2+}$ und $Fe^{2+}$) in Figur 4 gezeigt sind. Die Messungen wurden bei Konzentrationen von Calconcarbonsäure bzw. Calmagit und Ionenkonzentrationen von jeweils 0,0001M (außer für $Ca^{2+}$, hier 0,001 M) durchgeführt. Der pH-Wert wurde mit einer 0,5 M Ammonium/Ammoniak-Pufferlösung auf pH = 10 eingestellt. Die Figuren 3 und 4 lassen erkennen, dass die Absorptionskurven der Erkennungsstoff-Ionen-Assoziate sich im Vergleich zu den reinen Erkennungsstoffen verschieben und die jeweiligen Absorptionskurven unterschiedliche Kurvenmerkmale aufweisen. Schon auf dieser Basis lassen sich die genannten Kationen qualitativ differenzieren. Dies kann beispielsweise auch durch Abgleichen der jeweiligen Kurven mit Datenbank oder Literaturdaten erfolgen.

Nachfolgend sei anhand einer beispielhaften Berechnung/Herleitung eine exemplarische Möglichkeit zur quantitativen Bestimmung von Kationen (hier $Cu^{2+}$) mit einem erfindungsgemäßen Analysesystem bzw. Analyseverfahren unter Verwendung von Calmagit als Erkennungsstoff beschrieben.

Aus dem Stand der Technik ist die "Rank Annihilation Factor Analysis (RAFA)" Methode zur Analyse spektroskopischer Daten wie der der UV-Vis Spektroskopie bekannt. So können beispielsweiße Metallindikatoren (Erkennungsstoffe) genauer untersucht werden und deren Komplexbildungskonstanten mit einem bestimmten Metall, aber auch die experimentell nicht direkt zugänglichen Extinktionskoeffizienten der gebildeten Metall-Indikator-Komplexe bestimmt werden. In diesem Beispiel müsste zunächst ein Modell erstellt werden, welches aus den beiden genannten Konstanten (Komplexbildungskonstante, Extinktionskoeffizient), die Absorbanz von Proben mit bestimmten Anfangskonzentrationen an Indikator und Metallsalz errechnet. Danach werden reale Proben mit den im Modell genutzten Konzentrationen gemessen. Die im Modell eingesetzten Konstanten werden variiert und das Ergebnis des Modelles mit den realen Daten verglichen. Der Punkt mit der geringsten relativen Standardabweichung zwischen Probe und Modell gibt in diesem Beispiel das Optimum für die Komplexbildungskonstante und den Extinktionskoeffizienten wieder.

Ionenfluss in Systemen wie dem hiesig beschriebenen lässt sich durch die Nernst-Planck-Gleichung beschreiben:

$$J_i = -D_i * \nabla c_i - z_i * u_i * F * c_i * \nabla \phi$$

*(Gleichung 3)*

$J_i$ beschreibt hierbei den Ionenfluss des Ions i an dieser Stelle. $D_i$ ist der Diffusionskoeffizient, $c_i$ die Konzentration an der betrachteten Stelle, $z_i$ die Ladungszahl und $u_i$ die Mobilität von *i*, $\phi$ beschreibt das elektrische Potential. Der erste Term modelliert hierbei die Diffusion und der zweite den Ionenfluss durch die in der Zelle angelegte Spannung.

Für die Bestimmung der Extinktionskoeffizienten der genutzten Metall-Indikator-Komplexe, sowie deren Komplexbildungskonstanten, kann eine RAFA-ähnliche Methode verwendet werden. Hierfür sei zunächst angenommen, dass zwischen Metallion ($M^{2+}$) und Indikator ($Ind^{2-}$) ausschließlich folgende Reaktion stattfindet:

$$M^{2+} + Ind^{2-} \rightleftharpoons [M(Ind)]$$

Der gebildete Komplex wird im Folgenden mit M-Ind abgekürzt. Für diese Reaktion lässt sich nun das Massenwirkungsgesetz mit der Komplexbildungskonstante $K_B$ aufstellen:

$$K_B = \frac{[M - Ind]}{[M^{2+}] * [Ind^{2-}]}$$

*(Gleichung 4)*

**Ferner gelten** für die Konzentrationen im Gleichgewicht folgende Abhängigkeiten zu den Ursprungskonzentrationen $c_i$:

$$[M^{2+}] = c_{M^{2+}} - [M - Ind]$$

(Gleichung 5)

$$[Ind^{2-}] = c_{Ind^{2-}} - [M - Ind]$$

(Gleichung 6)

Im Folgenden wird [M-Ind] mit x abgekürzt. Durch Einsetzen von Gleichung 5 und 6 in Gleichung 4 und Umstellen ergibt sich Gleichung 7:

$$K_B * c_{Ind^{2-}} * c_{M^{2+}} - (K_B * (c_{Ind^{2-}} + c_{M^{2+}}) + 1) * x + K_B * x^2 = 0$$

(Gleichung 7)

Durch Lösen der Gleichung7 wird Gleichung 8 erhalten. Hierbei ist nur die Lösung mit einem negativen Vorzeichen vor der Wurzel sinnvoll.

$$x = \frac{K_B * (c_{Ind^{2-}} + c_{M^{2+}}) + 1 - \sqrt{(K_B * (c_{Ind^{2-}} + c_{M^{2+}}) + 1)^2 - 4 * K_B^2 * c_{Ind^{2-}} * c_{M^{2+}}}}{2 * K_B}$$

(Gleichung 8)

Für die Gesamtabsorbanz $A_{ges}$ der Probe wird angenommen, dass folgende Gleichung gilt:

$$A_{\text{ges}} = A_{\text{Ind}^{2-}} + A_{\text{M}^{2+}} + A_{\text{M-Ind}} + A_0$$

(Gleichung 9)

$A_0$ ist hierbei die Absorbanz bei der Messung des reinen Lösungsmittels. Durch Einsetzen des Lambert-Beer-Gesetzes und den Gleichungen 5 und 6 wird nun folgende Beziehung erhalten:

$$A_{\text{ges}} = (c_{\text{Ind}^{2-}} - x) * \varepsilon_{\text{Ind}^{2-}} + (c_{\text{M}^{2+}} - x) * \varepsilon_{\text{M}^{2+}} + x * \varepsilon_{\text{M-Ind}} + A_0$$

(Gleichung 10)

Die Küvettenlänge kann durch eine Pathlength-Correction auf 1 cm normiert werden, sodass diese zur Vereinfachung weggelassen werden kann. Die Extinktionskoeffizienten für das reine Salz $\varepsilon_M{}^{2+}$ sowie für den reinen Indikator $\varepsilon_{Ind}{}^{2-}$ lassen sich direkt über das Lambert-Beer-Gesetz aus Absorbanzen reiner Lösungen dieser Stoffe bestimmen. $A_{ges}$ wiederum lässt sich durch die Messung einer realen Lösung mit $c_{Ind}{}^{2-}$ und $\varepsilon_M{}^{2+}$ bestimmen. Da *x* über Gleichung 8 zugänglich ist, kann nun mit diesen Daten und durch Umstellen von Gleichung 10 der nicht zugängliche Extinktionskoeffizient des Metall-Indikator-Komplexes $\varepsilon_{M\text{-}Ind}$ bestimmt werden:

$$\varepsilon_{\text{M-Ind}} = \frac{A_{ges} - (c_{\text{Ind}^{2-}} - x) * \varepsilon_{\text{Ind}^{2-}} - (c_{\text{M}^{2+}} - x) * \varepsilon_{\text{M}^{2+}} - A_0}{x}$$

(Gleichung 11)

**Um nun** $K_B$ **und** $\varepsilon_{M\text{-}Ind}$ bestimmen zu können, können Proben mit verschiedenen $c_{Ind}{}^{2-}$ und $\varepsilon_M{}^{2+}$ gemessen werden. Im Anschluss kann $K_B$ über einen breiten Wertebereich variiert und jeweils $\varepsilon_{M\text{-}Ind}$ für die einzelnen Proben bestimmt werden. Für jedes eingesetzte $K_B$ kann die relative Standardabweichung $\sigma_R$ unter den $\varepsilon_{M\text{-}Ind}$ mit folgender Formel bestimmt:

$$\sigma_R = \sqrt{\frac{\sum_{i=1}^{n} (\varepsilon_{\text{M-Ind},i} - \widehat{\varepsilon_{\text{M-Ind}}})^2}{n-1}} * \frac{1}{\widehat{\varepsilon_{\text{M-Ind}}}}$$

(Gleichung 12)

Der optimale Wert für $K_e$ wird bei minimalem $\sigma_R$ erhalten. Der optimale Wert für $\varepsilon_{M\text{-}Ind}$ wird an dieser Stelle aus dem Mittelwert der einzelnen $\varepsilon_{M\text{-}Ind}$ der einzelnen Proben bestimmt. Für die Untersuchung von Metall-Indikator-Komplexen mit zwei Indikatorliganden pro Metallion gelten analoge Gleichungen, ausgehend von folgendem Massenwirkungsgesetz:

$$K_B = \frac{[\text{M} - 2\text{Ind}]}{[\text{M}^{2+}] * [\text{Ind}^{2-}]^2}$$

(Gleichung 13)

Problematisch für die Berechnung der Metallionenkonzentrationen in der Messkammer (hier der zweiten Messkammer für Kationen), ist die Tatsache, dass sich die Absorbanzen des ungebundenen Calmagits sowie die der Metall-Calmagit-Komplexe überlagern. Um dieses Problem zu lösen, wird Gleichung 9 erweitert und angenommen, dass die nicht im Komplex gebundenen Metallionen kaum absorbieren.

$$A_{\mathrm{ges},\lambda} = A_{\mathrm{Ind}^{2-},\lambda} + \sum A_{\mathrm{M}_i-\mathrm{Ind},\lambda} + A_{0,\lambda}$$

Gleichung (14)

$$A_{\mathrm{ges},\lambda} = \varepsilon_{\mathrm{Ind}^{2-},\lambda} * [\mathrm{Ind}^{2-}] + \sum \varepsilon_{\mathrm{M}_i-\mathrm{Ind}} * [\mathrm{M}_i - \mathrm{Ind}] + A_{0,\lambda}$$

Gleichung (15)

**Gleichung** 15 ergibt sich hierbei durch Einsetzen des Lambert-Beer-Gesetzes. Hierbei ist der Extinktionskoeffizient des Indikators $\varepsilon_{\mathrm{Ind}}{}^{2-}{}_{,\lambda}$ bei der Wellenlänge λ bereits aus den beschriebenen Messungen bekannt. Die Extinktionskoeffizienten der Metall-Indikator-Komplexe $\varepsilon_{\mathrm{M}_i\text{-Ind},\lambda}$ sind durch die Daten der Untersuchung der Metall-Indikator-Komplexe zugänglich, da hierbei die Konzentrationen der einzelnen Stoffe in der untersuchten Lösung bestimmt werden. Somit kann Gleichung 11 auch bei anderen Wellenlängen genutzt und die Konzentrationen eingesetzt werden. Um nun die einzelnen Konzentrationen in der Messzelle selbst bestimmen zu können, muss bei i+1 Wellenlängen die Absorbanz bestimmt werden. Da die Extinktionskoeffizienten der in Lösung vorliegenden Stoffe bei allen Wellenlängen bestimmbar sind, enthält man nun *i*+1 Gleichungen für die gleiche Anzahl unbekannter Konzentrationen. Durch Lösen dieses Gleichungssystems erhält man die Gleichgewichtskonzentrationen. Um nun die Gesamtkonzentrationen der Metallionen im System bestimmen zu können, werden die Gleichungen 4 und 5 genutzt:

$$c_{\mathrm{M},i} = [\mathrm{M}_i - \mathrm{Ind}] * \left(1 + \frac{1}{K_{\mathrm{B},i} * [\mathrm{Ind}^{2-}]}\right)$$

(Gleichung 16)

Die Berechnung der Konzentration der Kupferionen erfolgt über eine analoge Gleichung basierend auf Gleichung 13.

Zur Modellierung der Konzentration in den Messkammern müssen zunächst einige Annahmen getroffen werden. So muss angenommen werden, dass die Ionendiffusion durch die Membran vernachlässigbar ist. Zudem soll auch die Bildung von Grenzschichten zunächst nicht weiter berücksichtigt werden. Somit ergibt sich folgende vereinfachte Nernst-Planck-Gleichung für den Ionenfluss $J_i$ durch die Membrane:

$$J_i = -z_i * u_i * F * c_i * \nabla\phi$$

(Gleichung 17)

Nun wird angenommen, dass sich das Potential ϕ in der Lösung nur linear ändert und ∇ϕ somit konstant ist. Durch Umschreiben als Geschwindigkeitsgesetz erhält man nun folgende Gleichung:

$$-\frac{dc_a}{dt} = k * c_a$$

(Gleichung 18)

k beschreibt hierbei die vorkommenden Konstanten, $c_a$ beschreibt die Konzentration des jeweiligen Metallions außerhalb der Messkammer, also vor der Membran. Durch Integration wird nun folgende Gleichung erhalten:

$$\int_{c_{a,o}}^{c_{a,1}} c^{-1} * dc = -k * \int_0^t dt$$

(Gleichung 19)

Somit gilt:

$$c_{a,1} = c_{a,0} * e^{-k*t}$$

(Gleichung 20)

$$c_{a,1} - c_{a,0} = \Delta c_a$$

(Gleichung 21)

Zudem ist die Änderung der messbaren Konzentration in der Messkammer $\Delta c$ über das Volumen *V* dieser, sowie über das Volumen der äußeren Probe *Va,* von der Änderung der äußeren Konzentration $c_a$ abhängig, da die Ionen von der Probe in die Messkammer wandern. Somit ergibt sich folgende Gleichung:

$$\Delta c_a = -\Delta c * \frac{V}{V_a}$$

(Gleichung 22)

Durch Einsetzen der Gleichungen 21 und 22 in Gleichung 20, sowie anschließendes Umstellen wird nun ein Gesetz zur Bestimmung der anfänglichen Außenkonzentration bei bekannter Änderung der Innenkonzentration erhalten, womit letztlich die Konzentrationsbestimmung der Probe möglich ist:

$$c_{a,0} = \frac{\Delta c * \frac{V}{V_a}}{1 - e^{-k*t}}$$

(Gleichung 23)

**Patentansprüche**

**1.** Analysesystem zur qualitativen und/oder quantitativen Bestimmung von Ionen in einem ionenhaltigen flüssigen Medium (9), wobei die Ionen Kationen (K) und/oder Anionen (A) sind, das Analysesystem umfassend:

- eine Messzelle (1) umfassend: eine erste Messkammer (3), eine zweite Messkammer (4), sowie eine zwischen der ersten Messkammer (3) und der zweiten Messkammer (4) angeordnete Probenkammer (2), wobei die Probenkammer (2) zur Aufnahme des ionenhaltigen flüssigen Mediums (9) ausgebildet ist, wobei in der ersten Messkammer (3) eine erste Elektrode (5) und in der zweiten Messkammer (4) eine zweite Elektrode (6) angeordnet ist, und wobei zwischen der ersten und zweiten Elektrode (5, 6) eine Spannung anlegbar ist, wodurch die erste Elektrode (5) positiv und die zweite Elektrode (6) negativ polarisierbar ist, und wodurch die Anionen (A) in Richtung der ersten Elektrode (5) und die Kationen (K) in Richtung der zweiten Elektrode (6) beförderbar sind,

wobei zwischen der ersten Messkammer (3) und der Probenkammer (2) eine anionenselektive Membran (7)

sowie zwischen der zweiten Messkammer (4) und der Probenkammer (2) eine kationenselektive Membran (8) angeordnet ist, wobei die anionenselektive Membran (7) dazu ausgebildet ist, einen Durchtritt von Anionen (A) aus der Probenkammer (2) in die erste Messkammer (3) zu ermöglichen, und wobei die kationenselektive Membran (8) dazu ausgebildet ist, einen Durchtritt von Kationen (K) aus der Probenkammer (2) in die zweite Messkammer (4) zu ermöglichen,
wobei die erste Messkammer (3) mit einem ersten flüssigen Messkammermedium befüllt ist und wobei in dem ersten flüssigen Messkammermedium ein erster Erkennungsstoff (E-1) enthalten ist, um bei Durchtritt der Anionen (A) in die erste Messkammer (3) Anionen-Erkennungsstoff-Assoziate (A-E-1) zu bilden, und wobei die zweite Messkammer (4) mit einem zweiten flüssigen Messkammermedium befüllt ist und wobei in dem zweiten flüssigen Messkammermedium ein zweiter Erkennungsstoff (E-2) enthalten ist, um bei Durchtritt der Kationen (K) in die zweite Messkammer (4) Kationen-Erkennungsstoff-Assoziate (K-E-2) zu bilden,

sowie
- eine Messanordnung, die dazu ausgebildet ist,

a. im Wege einer photometrischen Messung die in der ersten Messkammer (3) vorliegenden Anionen (A) und/oder Anionen-Erkennungsstoff-Assoziate (A-E-1) photometrisch zu messen, und auf Basis dieser Messung(en) die Anionen (A) qualitativ und/oder quantitativ zu bestimmen, und/oder
b. im Wege einer photometrischen Messung die in der zweiten Messkammer (4) vorliegenden Kationen (K) und/oder Kationen-Erkennungsstoff-Assoziate (K-E-2) photometrisch zu messen, und auf Basis dieser Messung(en) die Kationen (K) qualitativ und/oder quantitativ zu bestimmen.

**2.** Analysesystem nach Anspruch 1, wobei in der ersten Messkammer (3) eine erste Mischvorrichtung angeordnet ist, um das in der ersten Messkammer (3) enthaltene erste flüssige Messkammermedium zu durchmischen, und/oder wobei in der zweiten Messkammer (4) eine zweite Mischvorrichtung angeordnet ist, um das in der zweiten Messkammer (4) enthaltene zweite flüssige Messkammermedium zu durchmischen.

**3.** Analysesystem nach Anspruch 1 oder 2, wobei, zur Einstellung eines pH-Wertes des in der ersten Messkammer (3) enthaltenen ersten flüssigen Messkammermediums und/oder des in der zweiten Messkammer (4) enthaltenen zweiten flüssigen Messkammermediums,

a. zwischen der ersten Messkammer (3) und der zweiten Messkammer (4) eine ladungsdurchlässige Membran angeordnet ist, um einen Ladungsdurchtritt zwischen der ersten Messkammer (3) und der zweiten Messkammer (4) zu ermöglichen, oder
b. eine erste ladungsdurchlässige Membran zwischen der ersten Messkammer (3) und einer mit einer ersten Wasserelektrolyselektrode beaufschlagten ersten Wasserelektrolysekammer angeordnet ist, um einen Ladungsdurchtritt zwischen der ersten Messkammer (3) und der ersten Wasserelektrolysekammer zu ermöglichen, und/oder eine zweite ladungsdurchlässige Membran zwischen der zweiten Messkammer (4) und einer mit einer zweiten Wasserelektrolyselektrode beaufschlagten zweiten Wasserelektrolysekammer angeordnet ist, um einen Ladungsdurchtritt zwischen der zweiten Messkammer (4) und der zweiten Wasserelektrolysekammer zu ermöglichen.

**4.** Analysesystem nach Anspruch 1, wobei die Messanordnung umfasst:

- eine Lichtquelle (10), die dazu ausgebildet ist, einen Lichtstrahl zu erzeugen,
- einen Messraum, wobei der Messraum

a. durch die erste Messkammer (3) und/oder die zweite Messkammer (4) ausgebildet ist, oder
b. extern zur Messzelle (1) angeordnet ist, wobei der Messraum mit dem ersten und/oder zweiten flüssigen Messkammermedium befüllbar ist,

- einen zwischen der Lichtquelle (10) und dem Messraum angeordneten ersten Lichtwellenleiter (12a), der dazu eingerichtet ist, den Lichtstrahl von der Lichtquelle (10) zu dem Messraum weiterzuleiten, und einen zwischen dem Messraum und einem Detektor (13) angeordneten zweiten Lichtwellenleiter (12b), der dazu eingerichtet ist, den Lichtstrahl von dem Messraum zu dem Detektor (13) weiterzuleiten, wobei der Detektor (13) dazu ausgebildet ist, einen transmittierten Anteil des Lichtstrahls nach Durchgang des Lichtstrahls durch im Messraum befindliches erstes oder zweites flüssiges Messkammermedium zu detektieren, und
- eine Auswerteeinheit (14), die dazu ausgebildet ist, auf Basis eines von dem Detektor (13) erhaltenen Signals,

die in dem ionenhaltigen flüssigen Medium (9) vorliegenden Kationen und/oder Anionen qualitativ und/oder quantitativ zu bestimmen.

**5.** Analysesystem nach Anspruch 4, wobei die Lichtquelle (10), der Detektor (13) und die Auswerteeinheit (14) extern zur Messzelle (1) angeordnet sind.

**6.** Analysesystem nach Anspruch 1, wobei die Probenkammer (2) einen Einlass zur Aufnahme des ionenhaltigen flüssigen Mediums (9) aufweist, wobei die Probenkammer (2)

a. durch den Einlass mit dem ionenhaltigen flüssigen Medium (9) befüllbar ist, oder
b. von dem ionenhaltigen flüssigen Medium (9) über den Einlass in Richtung eines Auslasses durchströmbar ist, wobei das Analysesystem ferner umfasst:

- ein Probenreservoir (18) für das ionenhaltige flüssige Medium (9), das über ein flüssigkeitsleitendes Bauteil (17) mit dem Einlass der Probenkammer (2) verbunden ist,
- eine Pumpeinheit (16), die dazu ausgebildet ist, das ionenhaltige flüssige Medium (9) aus dem Probenreservoir (18) über das flüssigkeitsleitende Bauteil (17) durch die Probenkammer (2) zu pumpen.

**7.** Analysesystem nach Anspruch 6, ferner umfassend eine zwischen der Pumpeinheit (16) und dem Einlass angeordnete Filtereinheit, um das ionenhaltige flüssige Medium (9) zu filtern.

**8.** Analysesystem nach einem der vorhergehenden Ansprüche, wobei die Elektroden (5, 6) aus demselben Metall oder verschiedenen Metallen, beispielsweise Titan oder Platin, gefertigt sind.

**9.** Analysesystem nach einem der vorhergehenden Ansprüche, wobei der erste Erkennungsstoff (E-1) und/oder der zweite Erkennungsstoff (E-2) ausgewählt ist aus der Gruppe: Calmagit, Dimethylglyoxim, Calconcarbonsäure, Xylenolorange, Eriochromschwarz T, Eriochromblauschwarz R, Ethylendiamintetraacetat (EDTA), Oxalsäure, Kalignost, Murexid, Methylthymolblau, Metallphthalein, Brenzcatechinviolett, 1-(2-Pyridylazo)-2-naphthol, 4-(2-Pyridylazo)resorcin, Eisen(III)chlorid, oder Mischungen davon.

**10.** Analysesystem nach einem der vorhergehenden Ansprüche, wobei die Kationen ausgewählt sind aus der Gruppe: Eisen(II)-Ionen, Eisen(III)-Ionen, Kupfer(II)-Ionen, Magnesium(II)-Ionen, Calcium(II)-Ionen, Mangan(II)-Ionen, Kalium(I)-Ionen, Ammoniumkationen ($NH_4^+$), oder Mischungen daraus, und/oder wobei die Anionen ausgewählt sind aus der Gruppe: Nitrat-Ionen ($NO_3^-$), Nitrit-Ionen ($NO_2^-$), Phosphat-Ionen ($PO_4^{3-}$), oder Mischungen daraus.

**11.** Analysesystem nach einem der vorhergehenden Ansprüche, wobei das erste und/oder zweite flüssige Messkammermedium einen Elektrolyten, vorzugsweise Natriumsulfat, umfasst.

**12.** Analysesystem nach einem der vorhergehenden Ansprüche, wobei die anionenselektive Membran (7) und/oder die kationenselektive Membran (8) jeweils aus einem Membranmaterial gefertigt ist, das umfasst:

- ein erstes Polymermaterial, vorzugsweise modifiziertes Polystyrol (PS), mit an einer Polymermaterialoberfläche angeordneten funktionellen Gruppen, wobei die an der Polymermaterialoberfläche angeordneten funktionellen Gruppen eine Anionenselektivität der anionenselektiven Membran (7) und/oder eine Kationenselektivität der kationenselektiven Membran (8) bereitstellen,
wobei die an der Polymermaterialoberfläche angeordneten funktionellen Gruppen zur Bereitstellung der Anionenselektivität der anionenselektiven Membran (7) vorzugsweise quaternäre Amine sind, und/oder wobei die an der Polymermaterialoberfläche angeordneten funktionellen Gruppen zur Bereitstellung der Kationenselektivität der kationenselektiven Membran (8) vorzugsweise Carbonsäure- oder Sulfonsäuregruppen sind,
- ein zweites Polymermaterial zur mechanischen Verstärkung der anionenselektiven Membran (7) und/oder der kationenselektiven Membran (8), vorzugsweise Polyvinylchlorid (PVC), Polypropylen (PP) oder Polyethylenterephthalat (PET).

**13.** Analyseverfahren zur qualitativen und/oder quantitativen Bestimmung von Ionen in einem ionenhaltigen flüssigen Medium (9) mit dem Analysesystem gemäß einem der Ansprüche 1-12, umfassend die folgenden Schritte:

- S0-a: Anlegen einer Spannung zwischen der ersten Elektrode (5) und der zweiten Elektrode (6), wodurch die erste Elektrode (5) positiv und die zweite Elektrode (6) negativ polarisiert wird, und wodurch Anionen (A) von der

Probenkammer (2) durch die anionenselektive Membran (7) in Richtung der in der ersten Messkammer (3) angeordneten ersten Elektrode (5) und Kationen (K) von der Probenkammer (2) durch die kationenselektive Membran (8) in Richtung der in der zweiten Messzelle (4) angeordneten zweiten Elektrode (6) befördert werden.
- S1: Bilden von Anionen-Erkennungsstoff-Assoziaten (A-E-1) aus in der ersten Messkammer (3) befindlichen Anionen (A) und dem ersten Erkennungsstoff (E-1), und/oder Bilden von Kationen-Erkennungsstoff-Assoziaten (K-E-2) aus in der zweiten Messkammer (4) befindlichen Kationen (K) und dem zweiten Erkennungsstoff (E-2),
- S2: photometrisches Messen von in der ersten Messkammer (3) befindlichen Anionen (A) und/oder Anionen-Erkennungsstoff-Assoziaten (A-E-1) mit der Messanordnung, und/oder photometrisches Messen von in der zweiten Messkammer (4) befindlichen Kationen (K) und/oder Kationen-Erkennungsstoff-Assoziaten (K-E-2) mit der Messanordnung,
- S3: qualitatives und/oder quantitatives Bestimmen der Anionen (A) und/oder Kationen (K) auf Basis der photometrischen Messung in Schritt S2.

**14.** Analyseverfahren nach Anspruch 13, wobei vorgelagert zum optionalen Schritt S1, oder vorgelagert zum Schritt S2, folgender Schritt ausgeführt wird:

- S0-b: Einstellen eines pH-Wertes des in der ersten Messkammer (3) enthaltenen ersten flüssigen Messkammermediums und/oder des in der zweiten Messkammer (4) enthaltenen zweiten flüssigen Messkammermediums mit einem Analysesystem nach Anspruch 3.

**15.** Analyseverfahren nach Anspruch 13, wobei nach dem Schritt S3, oder vorgelagert zum Schritt S0-a, folgender Schritt ausgeführt wird:

- S4: Anlegen einer im Vergleich zu der in Schritt S0-a angelegten, umgekehrten Spannung zwischen der ersten Elektrode (5) und der zweiten Elektrode (6), wodurch die erste Elektrode (5) negativ und die zweite Elektrode (6) positiv polarisiert wird,

wodurch die im optionalen Schritt S1 in der ersten Messkammer (3) gebildeten Anionen-Erkennungsstoff-Assoziate (A-E-1) zu Anionen (A) und dem ersten Erkennungsstoff (E-1) zurückgebildet werden und Anionen (A) von der ersten Messkammer (3) durch die anionenselektive Membran (7) in die Probenkammer (2) zurückbefördert werden, wodurch der erste Erkennungsstoff (E-1) regeneriert wird, und/oder
wodurch die im optionalen Schritt S1 in der zweiten Messkammer (4) gebildeten Kationen-Erkennungsstoff-Assoziate (K-E-2) zu Kationen (K) und dem zweiten Erkennungsstoff (E-2) zurückgebildet werden und Kationen (K) von der zweiten Messkammer (4) durch die kationenselektive Membran (8) in die Probenkammer (2) zurückbefördert werden, wodurch der zweite Erkennungsstoff (E-2) regeneriert wird.

**16.** Analysesystem nach einem der Ansprüche 1-12, wobei das Analysesystem als eine kompakte Sonde mit integrierter Pumpeinheit unmittelbar in einem Probenreservoir angeordnet ist.

## Claims

**1.** An analysis system for the qualitative and/or quantitative determination of ions in an ion-containing liquid medium (9), wherein the ions are cations (K) and/or anions (A), the analysis system comprising:

- a measuring cell (1) comprising: a first measuring chamber (3), a second measuring chamber (4), and a sample chamber (2) arranged between the first measuring chamber (3) and the second measuring chamber (4), wherein the sample chamber (2) is designed to accommodate the ion-containing liquid medium (9), wherein a first electrode (5) is arranged in the first measuring chamber (3) and a second electrode (6) is arranged in the second measuring chamber (4), and wherein a voltage can be applied between the first and second electrodes (5, 6), whereby the first electrode (5) can be positively polarised and the second electrode (6) can be negatively polarised, and whereby the anions (A) can be transported towards the first electrode (5) and the cations (K) can be transported towards the second electrode (6),

wherein an anion-selective membrane (7) is arranged between the first measuring chamber (3) and the sample chamber (2), and a cation-selective membrane (8) is arranged between the second measuring chamber (4) and the sample chamber (2), wherein the anion-selective membrane (7) is designed to enable a passage of anions (A) out of the sample chamber (2) into the first measuring chamber (3), and wherein the

cation-selective membrane (8) is designed to enable a passage of cations (K) out of the sample chamber (2) into the second measuring chamber (4),
wherein the first measuring chamber (3) is filled with a first liquid measuring chamber medium, and wherein optionally a first detection substance (E-1) is contained in the first liquid measuring chamber medium, in order to form anion detection substance associates (A-E-1) when anions (A) pass into the first measuring chamber (3), and
wherein the second measuring chamber (4) is filled with a second liquid measuring chamber medium, and wherein optionally a second detection substance (E-2) is contained in the second liquid measuring chamber medium, in order to form cation detection substance associates (K-E-2) when cations (K) pass into the second measuring chamber (4),

and
- a measuring arrangement that is designed

a. to measure the anions (A) and/or anion detection substance associates (A-E-1) present in the first measuring chamber (3) photometrically by way of a photometric measurement, and to determine the anions (A) qualitatively and/or quantitatively on the basis of this/these measurement(s), and/or
b. to measure the cations (K) and/or cation detection substance associates (K-E-2) present in the second measuring chamber (4) photometrically by way of a photometric measurement, and to determine the cations (K) qualitatively and/or quantitatively on the basis of this/these measurement(s).

**2.** The analysis system according to claim 1, wherein a first mixing apparatus is arranged in the first measuring chamber (3) to mix the first liquid measuring chamber medium contained in the first measuring chamber (3), and/or wherein a second mixing apparatus is arranged in the second measuring chamber (4) to mix the second liquid measuring chamber medium contained in the second measuring chamber (4).

**3.** The analysis system according to claim 1 or 2, wherein in order to adjust a pH of the first liquid measuring chamber medium contained in the first measuring chamber (3) and/or of the second liquid measuring chamber medium contained in the second measuring chamber (4),

a. a charge-permeable membrane is arranged between the first measuring chamber (3) and the second measuring chamber (4) to enable a charge to pass between the first measuring chamber (3) and the second measuring chamber (4), or
b. a first charge-permeable membrane is arranged between the first measuring chamber (3) and a first water electrolysis chamber fitted with a first water electrolysis electrode, to enable a charge to pass between the first measuring chamber (3) and the first water electrolysis chamber, and/or a second charge-permeable membrane is arranged between the second measuring chamber (4) and a second water electrolysis chamber fitted with a second water electrolysis electrode to enable a charge to pass between the second measuring chamber (4) and the second water electrolysis chamber.

**4.** The analysis system according to claim 1, wherein the measuring arrangement comprises:

- a light source (10) that is designed to generate a light beam,
- a measuring space, wherein the measuring space

**a.** is formed by the first measuring chamber (3) and/or the second measuring chamber (4), or
b. is arranged outside the measuring cell (1), wherein the measuring space can be filled with the first and/or second liquid measuring chamber medium,

- a first optical waveguide (12a) arranged between the light source (10) and the measuring space, which waveguide is configured to guide the light beam from the light source (10) to the measuring space, and a second optical waveguide (12b) arranged between the measuring space and a detector (13), which waveguide is configured to guide the light beam from the measuring space to the detector (13), wherein the detector (13) is designed to detect a transmitted component of the light beam after the light beam has passed through first or second liquid measuring chamber medium located in the measuring space, and
- an evaluation unit (14), which is designed to determine cations and/or anions present in the ion-containing liquid medium (9) qualitatively and/or quantitatively on the basis of a signal received from the detector (13).

**5.** The analysis system according to claim 4, wherein the light source (10), the detector (13) and the evaluation unit (14) are arranged outside the measuring cell (1).

**6.** The analysis system according to claim 1, wherein the sample chamber (2) has an inlet for receiving the ion-containing liquid medium (9), wherein the sample chamber (2)

**a.** can be filled with the ion-containing liquid medium (9) through the inlet, or
**b.** the ion-containing liquid medium (9) can flow through the sample chamber from the inlet towards an outlet, wherein the analysis system further comprises:

- a sample reservoir (18) for the ion-containing liquid medium (9), which is connected to the inlet of the sample chamber (2) via a liquid-carrying component (17),
- a pump unit (16) which is designed to pump the ion-containing liquid medium (9) out of the sample reservoir (18) and through the sample chamber (2) via the liquid-conducting component (17).

**7.** The analysis system according to claim 6, further comprising a filter unit arranged between the pump unit (16) and the inlet in order to filter the ion-containing liquid medium (9) .

**8.** The analysis system according to any one of the preceding claims, wherein the electrodes (5, 6) are made from the same metal or different metals, for example titanium or platinum.

**9.** The analysis system according to any one of the preceding claims, wherein the first detection substance (E-1) and/or the second detection substance (E-2) is selected from the group: calmagite, dimethylglyoxime, calcon carboxylic acid, xylenol orange, eriochrome black T, eriochrome blue black R, ethylenediamine tetraacetate (EDTA), oxalic acid, Kalignost, murexide, methylthymol blue, metal phthalein, pyrocatechol violet, 1-(2-pyridylazo)-2-naphthol, 4-(2-pyridylazo) resorcinol, iron (III) chloride, or mixtures thereof.

**10.** The analysis system according to any one of the preceding claims, wherein the cations are selected from the group: iron(II) ions, iron(III) ions, copper (II) ions, magnesium (II) ions, calcium (II) ions, manganese (II) ions, potassium(I) ions, ammonium cations ($NH_4^+$), or mixtures thereof, and/or wherein the anions as selected from the group: nitrate ions ($NO_3^-$), nitrite ions ($NO_2^-$), phosphate ions ($PO_4^{3-}$), or mixtures thereof.

**11.** The analysis system according to any one of the preceding claims, wherein the first and/or second liquid measuring chamber medium comprises an electrolyte, preferably sodium sulfate.

**12.** The analysis system according to any one of the preceding claims, wherein the anion-selective membrane (7) and/or the cation-selective membrane (8) are each made from a membrane material that comprises:

- a first polymer material, preferably modified polystyrene (PS), with functional groups arranged on a polymer material surface, wherein the functional groups arranged on the polymer material surface functional groups lend an anion selectivity to the anion-selective membrane (7) and/or a cation selectivity to the cation-selective membrane (8),
wherein the functional groups arranged on the polymer material surface for lending anion selectivity to the anion-selective membrane (7) are preferably quaternary amines, and/or wherein the functional groups arranged on the polymer material surface for lending cation selectivity to the cation selective membrane (8) are preferably carboxylic acid or sulfonic acid groups,
- a second polymer material for mechanical strengthening of the anion-selective membrane (7) and/or the cation-selective membrane (8), preferably polyvinyl chloride (PVC), polypropylene (PP) or polyethylene terephthalate (PET).

**13.** An analysis process for qualitative and/or quantitative determination of ions in an ion-containing liquid medium (9) using the analysis system according to any one of claims 1-12, said process comprising the following steps:

- SO-a: application of a voltage between the first electrode (5) and the second electrode (6), whereby the first electrode (5) is positively polarised and the second electrode (6) is negatively polarised, and whereby anions (A) are transported from the sample chamber (2), through the anion-selective membrane (7) towards the first electrode (5) arranged in the first measuring chamber (3), and cations (K) are transported from the sample chamber (2), through the cation-selective membrane (8) towards the second electrode (6) arranged in the second

measuring cell (4),
- S1: forming anion detection substance associates (A-E-1) from anions (A) present in the first measuring chamber (3) and the first detection substance (E-1), and/or forming cation detection substance associates (K-E-2) from cations (K) present in the second measuring chamber (4) and the second detection substance (E-2),
- S2: photometric measurement of anions (A) and/or anion detection substance associates (A-E-1) present in the first measuring chamber (3) with the measuring arrangement, and/or photometric measurement of cations (K) and/or cation detection substance associates (K-E-2) present in the second measuring chamber (4) with the measuring arrangement,
- S3: qualitative and/or quantitative determination of the anions (A) and/or cations (K) on the basis of the photometric measurement in step S2.

**14.** The analysis process according to claim 13, wherein the following step is performed prior to the optional step S1, or prior to step S2:

- S0-b: adjustment of a pH of the first liquid measuring chamber medium contained in the first measuring chamber (3) and/or of the second liquid measuring chamber medium contained in the second measuring chamber (4) with an analysis system according to claim 3.

**15.** The analysis process according to claim 13, wherein the following step is performed after step S3 or prior to step SO-a:

- S4: application of a reverse voltage compared to the voltage applied in step S0-a between the first electrode (5) and the second electrode (6), whereby the first electrode (5) is negatively polarised and the second electrode (6) is positively polarised,
whereby the anion detection substance associates (A-E-1) formed in the first measuring chamber (3) in optional step S1 are reverted to anions (A) and the first detection substance (E-1), and anions (A) are transported from the first measuring chamber (3) through the anion-selective membrane (7) back into the sample chamber (2), whereby the first detection substance (E-1) is regenerated, and/or
whereby the cation detection substance associates (K-E-2) formed in the second measuring chamber (4) in optional step S1 are reverted to cations (K) and the second detection substance (E-2), and cations (K) are transported from the second measuring chamber (4) through the cation-selective membrane (8) back into the sample chamber (2), whereby the second detection substance (E-2) is regenerated.

**16.** The analysis system according to any one of claims 1-12, wherein the analysis system as a compact probe with integrated pump unit is arranged directly in a sample reservoir.

## Revendications

**1.** Système d'analyse, destiné à la détermination qualitative et / ou quantitative d'ions dans un milieu liquide (9) ionisé, les ions étant des cations (K) et / ou des anions (A), le système d'analyse comprenant :

- une cellule de mesure (1) comprenant : une première chambre de mesure (3), une deuxième chambre de mesure (4), ainsi qu'une chambre d'échantillonnage (2), placée entre la première chambre de mesure (3) et la deuxième chambre de mesure (4), la chambre d'échantillonnage (2) étant conçue pour recevoir le milieu liquide (9) ionisé, dans la première chambre de mesure (3) étant placée une première électrode (5) et dans la deuxième chambre de mesure (4) étant placée une deuxième électrode (6) et entre la première et la deuxième électrodes (5, 6) étant susceptible d'être appliquée une tension, suite à quoi, la première électrode (5) est polarisable positivement et la deuxième électrode (6) est polarisable négativement et suite à quoi les anions (A) sont transportables dans la direction de la première électrode (5) et les cations (K) sont transportables dans la direction de la deuxième électrode (6),

entre la première chambre de mesure (3) et la chambre d'échantillonnage (2) étant placée une membrane (7) sélectivement perméable aux anions et entre la deuxième chambre de mesure (4) et la chambre d'échantillonnage (2) étant placée une membrane (8) sélectivement perméable aux cations, la membrane (7) sélectivement perméable aux anions étant conçue pour permettre un passage d'anions (A) hors de la chambre d'échantillonnage (2) dans la première chambre de mesure (3) et la membrane (8) sélectivement perméable aux cations étant conçue pour permettre un passage de cations (K) hors de la chambre d'échantillonnage (2) dans la deuxième chambre de mesure (4),

la première chambre de mesure (3) étant remplie d'un premier milieu liquide de chambre de mesure et dans le premier milieu liquide de chambre de mesure étant contenue une première substance d'identification (E-1), pour constituer lors du passage des anions (A) dans la première chambre de mesure (3) des complexes de substances d'identification d'anions (A-E-1) et
la deuxième chambre de mesure (4) étant remplie d'un deuxième milieu liquide de chambre de mesure et dans le deuxième milieu liquide de chambre de mesure étant contenue une deuxième substance d'identification (E-2), pour constituer lors du passage des cations (K) dans la deuxième chambre de mesure (4) des complexes de substances d'identification de cations (K-E-2),

ainsi
- qu'un ensemble de mesure, qui est conçu pour

a. mesurer photométriquement par la voie d'une mesure photométrique les anions (A) et / ou les complexes de substances d'identification d'anions (A-E-1) présents dans la première chambre de mesure (3) et sur la base de ladite (desdites) mesure(s), déterminer qualitativement et / ou quantitativement les anions (A) et / ou
b. mesurer photométriquement par la voie d'une mesure photométrique les cations (K) et / ou les complexes de substances d'identification des cations (K-E-2) présents dans la deuxième chambre de mesure (4) et sur la base de ladite (desdites) mesures, déterminer qualitativement et / ou quantitativement les cations (K).

**2.** Système d'analyse selon la revendication 1, dans la première chambre de mesure (3) étant placé un premier dispositif mélangeur, destiné à mélanger intimement le premier milieu liquide de chambre de mesure contenu dans la première chambre de mesure (3) et / ou dans la deuxième chambre de mesure (4) étant placé un deuxième dispositif mélangeur, destiné à mélanger intimement le deuxième milieu liquide de chambre de mesure contenu dans la deuxième chambre de mesure (4).

**3.** Système d'analyse selon la revendication 1 ou 2, pour régler une valeur pH du premier milieu liquide de chambre de mesure contenu dans la première chambre de mesure (3) et / ou du deuxième milieu liquide de chambre de mesure contenu dans la deuxième chambre de mesure (4),

a. entre la première chambre de mesure (3) et la deuxième chambre de mesure (4) étant placée une membrane perméable à la charge, destinée à permettre un passage d'une charge entre la première chambre de mesure (3) et la deuxième chambre de mesure (4) ou
b. entre la première chambre de mesure (3) et une première chambre d'électrolyse de l'eau exposée à une première électrode d'électrolyse de l'eau étant placée une première membrane perméable à la charge, destinée à permettre un passage de la charge entre la première chambre de mesure (3) et la première chambre d'électrolyse de l'eau et / ou entre la deuxième chambre de mesure (4) et une deuxième chambre d'électrolyse de l'eau exposée à une deuxième électrode d'électrolyse de l'eau étant placée une deuxième membrane perméable à la charge, destinée à permettre un passage de la charge entre la deuxième chambre de (4) et la deuxième chambre d'électrolyse de l'eau.

**4.** Système d'analyse selon la revendication 1, l'ensemble de mesure comprenant :

- une source lumineuse (10), qui est conçue pour générer un faisceau lumineux,
- un espace de mesure, l'espace de mesure

a. étant conçu par la première chambre de mesure (3) et / ou par la deuxième chambre de mesure (4) ou
b. étant placé en externe par rapport à la cellule de mesure (1), l'espace de mesure étant susceptible d'être rempli avec le premier et / ou le deuxième milieu liquide de chambre de mesure,

- un premier conducteur à fibres optiques (12a), placé entre la source lumineuse (10) et l'espace de mesure, qui est configuré pour retransférer le faisceau lumineux de la source lumineuse (10) vers l'espace de mesure et un deuxième conducteur à fibres optiques (12b) placé entre l'espace de mesure et un détecteur (13), qui est configuré pour retransférer le faisceau lumineux de l'espace de mesure vers le détecteur (13), le détecteur (13) étant conçu pour détecter une part transmise du faisceau lumineux après le passage du faisceau lumineux à travers un premier ou un deuxième milieu liquide de chambre de mesure se trouvant dans l'espace de mesure et
- une unité d'évaluation (14), qui est conçue pour déterminer qualitativement et / ou quantitativement sur la base d'un signal reçu par le détecteur (13) les cations et / ou les anions présents dans le milieu liquide (9) ionisé.

5. Système d'analyse selon la revendication 4, la source lumineuse (10), le détecteur (13) et l'unité d'évaluation (14) étant placés en externe par rapport à la cellule de mesure (1).

6. Système d'analyse selon la revendication 1, la chambre d'échantillonnage (2) comportant une entrée, destinée à recevoir le milieu liquide (9) ionisé, la chambre d'échantillonnage (2)

   a. pouvant être remplie à travers l'entrée avec le milieu liquide (9) ionisé ou
   b. pouvant être traversée par le milieu liquide (9) par l'intermédiaire de l'entrée dans la direction d'une sortie, le système d'analyse comprenant par ailleurs :

      - un réservoir d'échantillonnage (18) pour le milieu liquide (9) ionisé, qui par l'intermédiaire d'un élément structurel (17) conducteur de liquide est relié avec l'entrée de la chambre d'échantillonnage (2),
      - une unité de pompage (16), qui est conçue pour pomper le milieu liquide (9) ionisé hors du réservoir d'échantillonnage (18), par l'intermédiaire de l'élément structurel (17) conducteur de liquide à travers la chambre d'échantillonnage (2).

7. Système d'analyse selon la revendication 6, comprenant par ailleurs une unité de filtration, placée entre l'unité de pompage (16) et l'entrée, pour filtrer le milieu liquide (9) ionisé.

8. Système d'analyse selon l'une quelconque des revendications précédentes, les électrodes (5, 6) étant fabriquées dans le même métal ou dans des métaux différents, par exemple en titane ou en platine.

9. Système d'analyse selon l'une quelconque des revendications précédentes, la première substance d'identification (E-1) et / ou la deuxième substance d'identification (E-2) étant sélectionnée dans le groupe comprenant : la calmagite, le diméthylglyoxime, l'acide calcone-carbonique, l'orange de xylénol, le noir ériochrome T, le bleu noir ériochrome R, l'éthylène diamine tétraacétique (EDTA), l'acide oxalique, le Kalignost (tétraphénlylborate de sodium), la murexide, le bleu de méthylthymol, le violet de phtaléine, le violet de catéchol, le 1-(2-pyridylazo)-2-naphtol, la 4-(2-pyridylazo) résorcine, le chlorure de fer(III) ou leurs mélanges.

10. Système d'analyse selon l'une quelconque des revendications précédentes, les cations étant sélectionnés dans le groupe comprenant : les ions de fer(II), les ions de fer(III), les ions de cuivre(II), les ions de magnésium(II), les ions de calcium(II),

    les ions de manganèse(II), les ions de potassium(I)-, les cations d'ammonium ($NH_4$+) ou leurs mélanges,
    et / ou les anions étant sélectionnés dans le groupe comprenant : les ions de nitrate ($NO_3^-$), les ions de nitrite ($NO_2^-$), les ions de phosphate ($PO_4^{3-}$) ou leurs mélanges.

11. Système d'analyse selon l'une quelconque des revendications précédentes, le premier et / ou le deuxième milieu liquide de chambre de mesure comprenant un électrolyte, de préférence un sulfate de sodium.

12. Système d'analyse selon l'une quelconque des revendications précédentes, la membrane (7) sélectivement perméable aux anions et / ou la membrane (8) sélectivement perméable aux cations étant fabriquée chacune dans une matière pour membrane, laquelle comprend :

    - une première matière polymère, de préférence un polystyrène modifié (PS), doté de groupes fonctionnels placés sur une surface de la matière polymère, les groupes fonctionnels placés sur la surface de la matière polymère mettant à disposition une perméabilité sélective aux anions de la membrane (7) sélectivement perméable aux anions et / ou une perméabilité sélective aux cations de la membrane (8) sélectivement perméable aux cations,
    les groupes fonctionnels placés sur la surface de la matière polymère destinés à mettre à disposition la perméabilité sélective aux anions de la membrane (7) sélectivement perméable aux anions étant de préférence des amines quaternaires et / ou les groupes fonctionnels placés sur la surface de la matière polymère destinés à mettre à disposition la perméabilité sélective aux cations de la membrane (8) sélectivement perméable aux cations étant de préférence des groupes d'acide carboxylique ou des groupes d'acide sulfonique,
    - une deuxième matière polymère, destinée à renforcer mécaniquement la membrane (7) sélectivement perméable aux anions et / ou la membrane (8) sélectivement perméable aux cations, de préférence un polychlorure de vinyle (PVC), un polypropylène (PP) ou un polytéréphtalate d'éthylène (PET).

**13.** Procédé d'analyse, destiné à la détermination qualitative et / ou quantitative d'ions dans un milieu liquide (9) ionisé à l'aide du système d'analyse selon l'une quelconque des revendications 1 à 12, comprenant les étapes suivantes consistant à :

- S0-a : appliquer une tension entre la première électrode (5) et la deuxième électrode (6), suite à quoi la première électrode (5) est polarisée positivement et la deuxième électrode (6) est polarisée négativement et suite à quoi, des anions (A) sont transportés de la chambre d'échantillonnage (2) à travers la membrane (7) sélectivement perméable aux anions dans la direction de la première électrode (5) placée dans la première chambre de mesure (3) et des cations (K) sont transportés de la chambre d'échantillonnage (2) à travers la membrane (8) sélectivement perméable aux cations dans la direction de la deuxième électrode (6) placée dans la deuxième cellule de mesure (4),
- S1 : constituer des substances d'identification d'anions (A-E-1) à partir d'anions (A) se trouvant dans la première chambre de mesure (3) et de la première substance d'identification (E-1) et / ou constituer des complexes de substances d'identification de cations (K-E-2) à partir de cations (K) se trouvant dans la deuxième chambre de mesure (4) et de la deuxième substance d'identification (E-2),
- S2 : à l'aide de l'ensemble de mesure, mesurer par photométrie des anions (A) et / ou des substances d'identification d'anions (A-E-1) se trouvant dans la première chambre de mesure (3) et / ou à l'aide de l'ensemble de mesure, mesurer par photométrie des cations (K) et / ou des complexes de substances d'identification de cations (K-E-2) se trouvant dans la deuxième chambre de mesure (4),
- S3 : déterminer qualitativement et / ou quantitativement les anions (A) et / ou les cations (K) sur la base de la mesure par photométrie réalisée à l'étape S2.

**14.** Procédé d'analyse selon la revendication 13, en amont de l'étape optionnelle S1 ou en amont de l'étape S2, étant réalisée l'étape suivante, consistant à :

- S0-b : régler une valeur pH pour le premier milieu liquide de chambre de mesure contenu dans la première chambre de mesure (3) et / ou pour le deuxième milieu liquide de chambre de mesure contenu dans la deuxième chambre de mesure (4), à l'aide d'un système d'analyse selon la revendication 3.

**15.** Procédé d'analyse selon la revendication 13, après l'étape S3 ou en amont de l'étape S0-a étant réalisée l'étape suivante consistant à

- S4 : appliquer une tension inverse en comparaison de celle appliquée à l'étape S0-a entre la première électrode (5) et la deuxième électrode (6), suite à quoi, la première électrode (5) est polarisée négativement et la deuxième électrode (6) est polarisée positivement,
suite à quoi, les complexes de substances d'identification d'anions (A-E-1) constitués lors de l'étape optionnelle S1 dans la première chambre de mesure (3) régressent en anions (A) et en la première substance d'identification (E-1) et des anions (A) sont transportés en retour de la première chambre de mesure (3) à travers la membrane (7) sélectivement perméable aux anions dans la chambre d'échantillonnage (2), suite à quoi, la première substance d'identification (E-1) se régénère et / ou
suite à quoi, les complexes de substances d'identification de cations (K-E-2) constitués lors de l'étape optionnelle S1 dans la deuxième chambre de mesure (4) régressent en cations (K) et en la deuxième substance d'identification (E-2) et des cations (K) sont transportés en retour de la deuxième chambre de mesure (4) à travers la membrane (8) sélectivement perméable aux cations dans la chambre d'échantillonnage (2), suite à quoi, la deuxième substance d'identification (E-2) se régénère.

**16.** Système d'analyse selon l'une quelconque des revendications 1 à 12, le système d'analyse consistant dans une sonde compacte dotée d'une unité de pompage intégrée étant placée directement dans un réservoir d'échantillonnage.

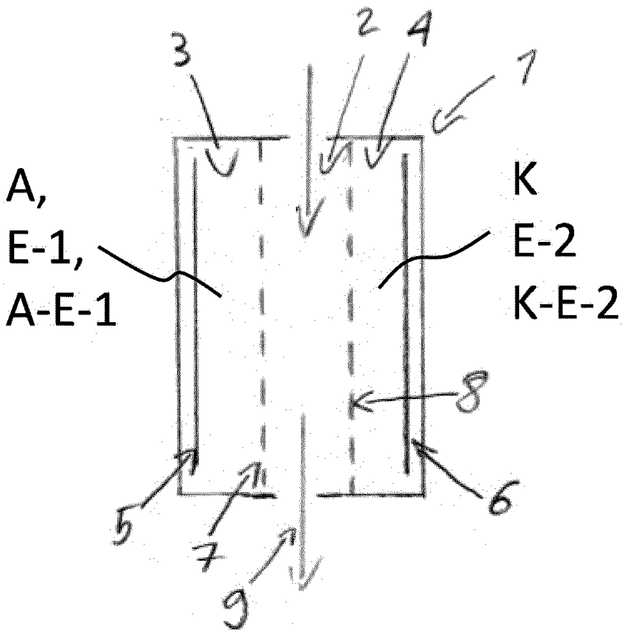


Fig. 1

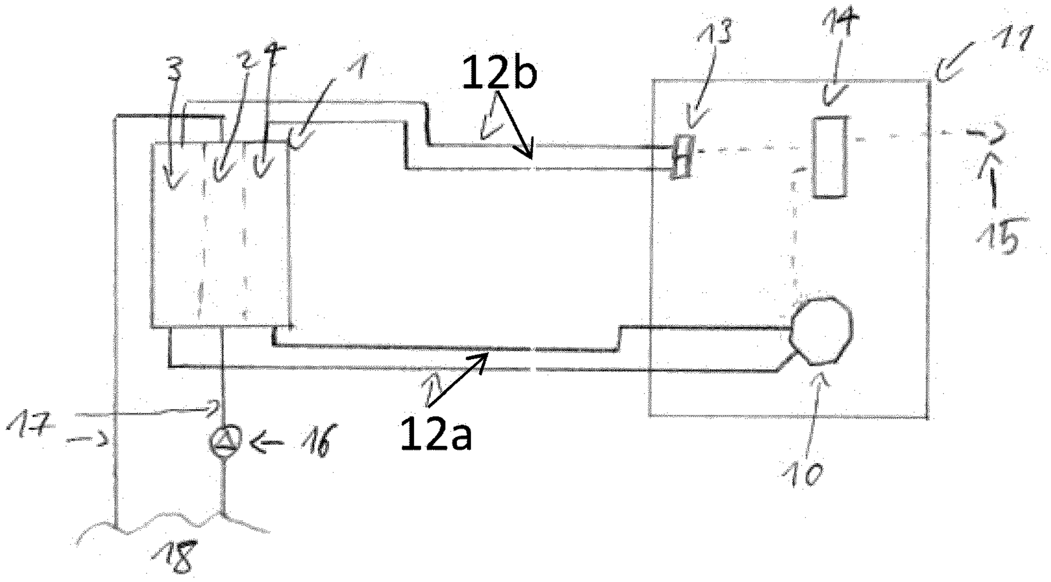


Fig. 2

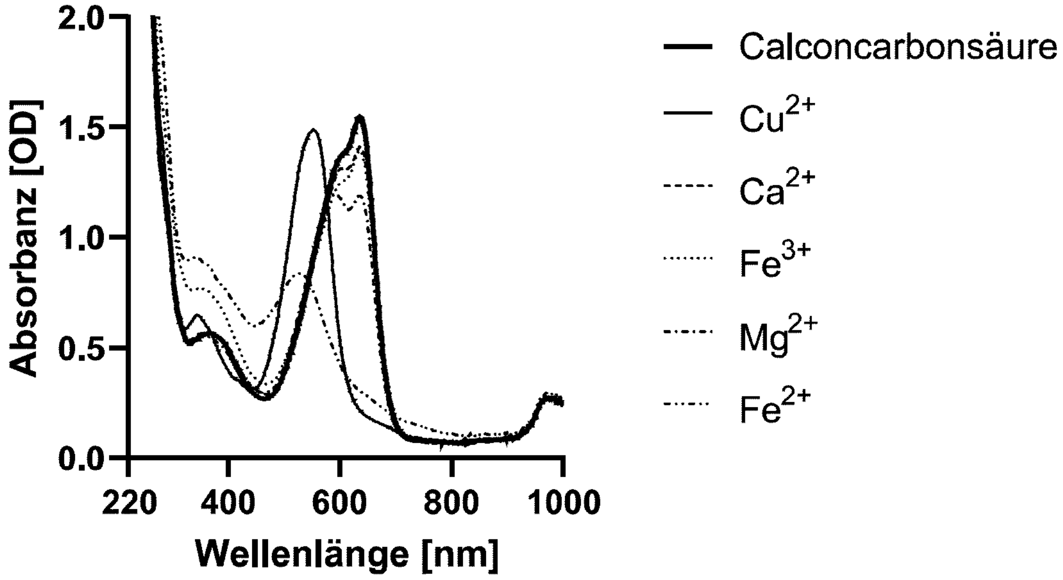
2.0
1.5
1.0
0.5
0.0
Absorbanz [OD]
220
400
600
800
1000
Wellenlänge [nm]
Calconcarbonsäure
Cu2+
Ca2+
Fe3+
Mg2+
Fe2+

Fig. 3

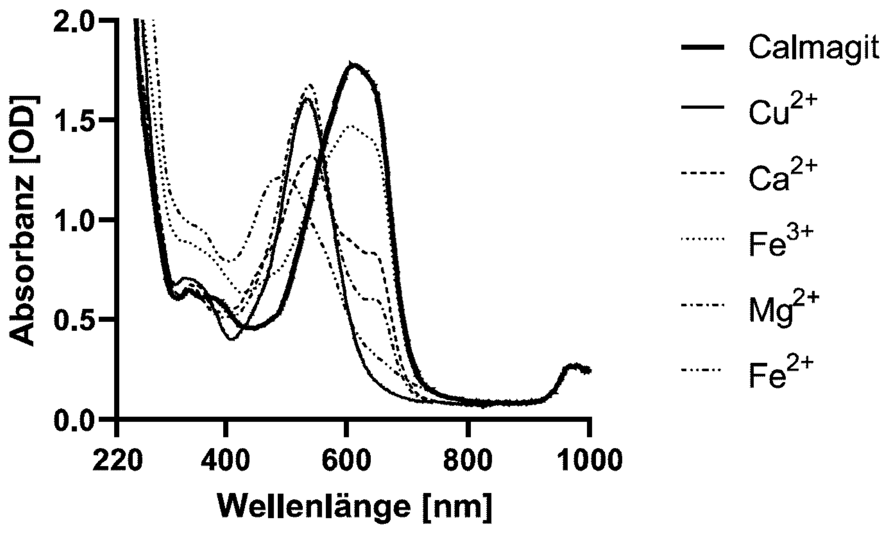
2.0
1.5
1.0
0.5
0.0
Absorbanz [OD]
220
400
600
800
1000
Wellenlänge [nm]
Calmagit
Cu2+
Ca2+
Fe3+
Mg2+
Fe2+

Fig. 4

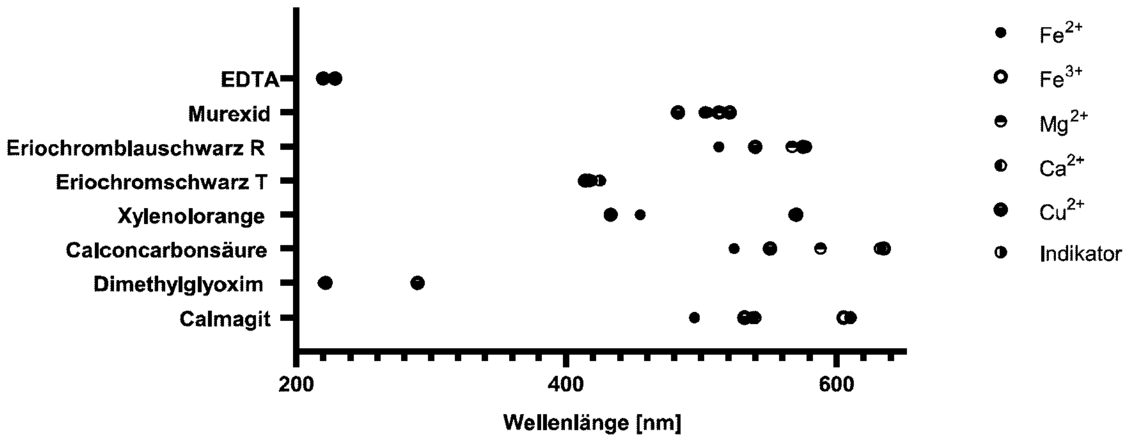
EDTA
Murexid
Eriochromblauschwarz R
Eriochromschwarz T
Xylenolorange
Calconcarbonsäure
Dimethylglyoxim
Calmagit
200
400
600
Wellenlänge [nm]
$Fe^{2+}$
$Fe^{3+}$
$Mg^{2+}$
$Ca^{2+}$
$Cu^{2+}$
Indikator

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP S579864 A


### In der Beschreibung aufgeführte Nicht-Patentliteratur


- *CHEMICAL ABSTRACTS*, 3147-14-6